# FASCICULE DE BREVET EUROPEEN

(11) **EP 2 185 547 B1**
(45) Date de publication et mention de la délivrance du brevet: **19.07.2017**
(21) Numéro de dépôt: 08838929.1
(22) Date de dépôt: 01.08.2008
(51) Int. Cl.: C07D 405/06, C07D 405/14, C07D 233/96, A61K 31/4166, A61K 31/4178

(54) **DERIVES D'IMIDAZOLONES, PROCEDE DE PREPARATION ET APPLICATIONS BIOLOGIQUES**
IMIDAZOLON-DERIVATE, VERFAHREN ZU IHRER HERSTELLUNG UND BIOLOGISCHE ANWENDUNG DAVON
IMIDAZOLONE DERIVATIVES, PREPARATION METHOD THEREOF AND BIOLOGICAL USE OF SAME

(30) Priorité: 01.08.2007 FR 0705632
(43) Date de publication de la demande: 19.05.2010
(73) Titulaire: Universite Rennes 1, 35065 Rennes Cédex (FR); Centre National de la Recherche Scientifique (C.N.R.S.), 75794 Paris Cedex 16 (FR)
(72) Inventeur: CARREAUX, François, F-35410 Châteaugiron (FR); BAZUREAU, Jean-Pierre, F-35520 La Chappelle des Fougerêtz (FR); RENAULT, Stéven, F-29120 Pont l'Abbé (FR); MEIJER, Laurent, F-29680 Roscoff (FR); LOZACH, Olivier, F-29860 Plabennec (FR)
(74) Mandataire: Nony
(86) Numéro de dépôt international: PCT/FR2008/001152
(87) Numéro de publication internationale: WO 2009/050352

(56) Documents cités:
- EP-A- 0 102 026
- EP-A- 0 422 900
- EP-A- 0 728 747
- EP-A- 1 484 051
- EP-A1- 0 057 882
- EP-A1- 0 449 216
- EP-A1- 0 565 135
- WO-A-2006/040052
- WO-A-2006/106046
- WO-A-2007/054508
- WO-A-2007/065261
- WO-A1-01/21166
- WO-A1-91/17151
- WO-A1-2004/052373
- WO-A2-2006/105237
- DE-B- 1 038 050
- JP-A- 2007 063 444
- DATABASE CA [Online] CHEMICAL ABSTRACTS SERVICE, COLUMBUS, OHIO, US; RAJPUROHIT, SANGEETA ET AL: "Synthesis and antimicrobial activity of 5'-aryl-substituted (4''-benzylidene-4,5-dihydro-5-oxo-1-(H)- -imidazolo)-1'',3'',4''- oxadiazoles" XP002479388 extrait de STN Database accession no. 2006:61023 & INDIAN JOURNAL OF HETEROCYCLIC CHEMISTRY , 15(2), 129-132 CODEN: IJCHEI; ISSN: 0971-1627, 2005,
- DATABASE CA [Online] CHEMICAL ABSTRACTS SERVICE, COLUMBUS, OHIO, US; SOLANKEE, ANJANI ET AL: "Synthesis and antimicrobial activity of 1-phenyl/substituted phenyl/benzyl/naphthyl-2-phenyl-4-(3-pheno xybenzylidene)imidazolin-5- ones" XP002479389 extrait de STN Database accession no. 2002:410854 & ASIAN JOURNAL OF CHEMISTRY , 14(2), 699-702 CODEN: AJCHEW; ISSN: 0970-7077, 2002,
- DATABASE CA [Online] CHEMICAL ABSTRACTS SERVICE, COLUMBUS, OHIO, US; AL-MADI, S. H. ET AL: "The in vitro antitumor assay of 5-(Z)-arylidene-4-imidazolidinones in screens of AIDS-related leukemia and lymphomas" XP002479390 extrait de STN Database accession no. 2002:30459 & ANTI-CANCER DRUGS , 12(10), 835-839 CODEN: ANTDEV; ISSN: 0959-4973, 2001,
- DATABASE CA [Online] CHEMICAL ABSTRACTS SERVICE, COLUMBUS, OHIO, US; CARTER, PERCY H. ET AL: "Photochemically enhanced binding of small molecules to the tumor necrosis factor receptor-1 inhibits the binding of TNF-.alpha.. [Erratum to document cited in CA136:95580]" XP002479391 extrait de STN Database accession no. 2002:9462 & PROCEEDINGS OF THE NATIONAL ACADEMY OF SCIENCES OF THE UNITED STATES OF AMERICA , 98(26), 15393 CODEN: PNASA6; ISSN: 0027-8424, 2001,
- DATABASE CA [Online] CHEMICAL ABSTRACTS SERVICE, COLUMBUS, OHIO, US; KHODAIR, AHMED I.: "Synthesis of 2-thiohydantoins and their S-glucosylated derivatives as potential antiviral and antitumor agents" XP002479392 extrait de STN Database accession no. 2001:710919 & NUCLEOSIDES, NUCLEOTIDES & NUCLEIC ACIDS , 20(9), 1735-1750 CODEN: NNNAFY; ISSN: 1525-7770, 2001,
- DATABASE CA [Online] CHEMICAL ABSTRACTS SERVICE, COLUMBUS, OHIO, US; CHEROUVRIER, JEAN RENE ET AL: "A stereoselective route to 3-methyl-2-methylsulfanyl-5-ylidene-3,5- dihydroimidazol-4-one derivatives and precursor of Leucettamine B" XP002479393 extrait de STN Database accession no. 2001:601202 & GREEN CHEMISTRY , 3(4), 165-169 CODEN: GRCHFJ; ISSN: 1463-9262, 2001,
- DATABASE CA [Online] CHEMICAL ABSTRACTS SERVICE, COLUMBUS, OHIO, US; SOLANKEE, ANJANI ET AL: "Synthesis and antimicrobial activity of 1-(phenyl- and substituted phenyl)-2-phenyl-4-(3',4',5'-trisubstitute d benzylidene)-5- imidazolones" XP002479394 extrait de STN Database accession no. 2000:391713 & ORIENTAL JOURNAL OF CHEMISTRY , 16(1), 155-158 CODEN: OJCHEG; ISSN: 0970-020X, 2000,
- DATABASE CA [Online] CHEMICAL ABSTRACTS SERVICE, COLUMBUS, OHIO, US; BHARATHI, K. ET AL: "Synthesis, pharmacological evaluation and QSAR studies of 4,5-dihydro-4-[(substituted phenyl) methylene]-5-oxo-2-phenyl/methyl- 1H-imidazole-1-acetic acids" XP002479395 extrait de STN Database accession no. 1999:669507 & INDIAN JOURNAL OF PHARMACEUTICAL SCIENCES , 61(3), 186-189 CODEN: IJSIDW; ISSN: 0250-474X, 1999,
- DATABASE CA [Online] CHEMICAL ABSTRACTS SERVICE, COLUMBUS, OHIO, US; SONI, V. C. ET AL: "Synthesis and antimicrobial activity of some new imidazolones having chloramphenicol base moiety" XP002479396 extrait de STN Database accession no. 1992:151653 & INDIAN JOURNAL OF PHARMACEUTICAL SCIENCES , 53(4), 185-7 CODEN: IJSIDW; ISSN: 0250-474X, 1991,
- DATABASE CA [Online] CHEMICAL ABSTRACTS SERVICE, COLUMBUS, OHIO, US; BOUSQUET, E. ET AL: "Synthesis and antiarrhythmic activity of 5-benzylidenimidazolin-4- one derivatives" XP002479397 extrait de STN Database accession no. 1979:468308 & BOLLETTINO CHIMICO FARMACEUTICO , 118(1), 23-7 CODEN: BCFAAI; ISSN: 0006-6648, 1979,
- DATABASE CA [Online] CHEMICAL ABSTRACTS SERVICE, COLUMBUS, OHIO, US; KUCHAR, M. ET AL: "Synthesis and antiinflammatory effect of 1- (ethoxycarbonylmethyl)imidazolin-5-one derivatives" XP002479398 extrait de STN Database accession no. 1976:446500 & CESKO-SLOVENSKA FARMACIE , 24(7), 287-92 CODEN: CKFRAY; ISSN: 0009-0530, 1975,
- DATABASE CA [Online] CHEMICAL ABSTRACTS SERVICE, COLUMBUS, OHIO, US; SAH, PRAMILLA ET AL: "Antimicrobial activity of some imidazolo substituted 4-thiazolidinones" XP002479399 extrait de STN Database accession no. 2007:318949 & INTERNATIONAL JOURNAL OF CHEMICAL SCIENCES , 4(4), 858-864 CODEN: IJCSIL; ISSN: 0972-768X, 2006,
- DATABASE CA [Online] CHEMICAL ABSTRACTS SERVICE, COLUMBUS, OHIO, US; SARAVANAN, SAMPATH ET AL: "Synthesis and antibacterial activity of some imidazole-5-(4H)one derivatives" XP002479400 extrait de STN Database accession no. 2005:1214648 & ARCHIV DER PHARMAZIE (WEINHEIM, GERMANY) , 338(10), 488-492 CODEN: ARPMAS; ISSN: 0365-6233, 2005,
- DATABASE CA [Online] CHEMICAL ABSTRACTS SERVICE, COLUMBUS, OHIO, US; GUPTA, J. K. ET AL: "Pharmacological evaluation of synthetic imidazolinones and their Schiff bases" XP002479401 extrait de STN Database accession no. 2005:966275 & INDIAN JOURNAL OF PHARMACEUTICAL SCIENCES , 67(3), 373-376 CODEN: IJSIDW; ISSN: 0250-474X, 2005,
- DATABASE CA [Online] CHEMICAL ABSTRACTS SERVICE, COLUMBUS, OHIO, US; KIEC-KONONOWICZ, KATARZYNA ET AL: "Preparation of novel 5-arylidene-4-oxo-2-imidazolidinylglycines as anticonvulsants" XP002479402 extrait de STN Database accession no. 2005:904548 & PL 187 019 B1 (UNIWERSYTET JAGIELLONSKI COLLEGIUM MEDICUM, POL.) 30 avril 2004 (2004-04-30)
- DATABASE CA [Online] CHEMICAL ABSTRACTS SERVICE, COLUMBUS, OHIO, US; SARAVANAN, V. S. ET AL: "Synthesis and antiinflammatory activity of some analogues of 4-substituted-2-phenyl imidazolin-5(4H)-one derivatives" XP002479403 extrait de STN Database accession no. 2005:38341 & ASIAN JOURNAL OF CHEMISTRY , 17(1), 576-580 CODEN: AJCHEW; ISSN: 0970-7077, 2005,
- DATABASE CA [Online] CHEMICAL ABSTRACTS SERVICE, COLUMBUS, OHIO, US; HOHARAM, HOSNY HAMED: "Process for the synthesis of 5-(4-dimethylaminobenzylidene)-2- phenylhydrazino[1,3-H]imidazole-4-one as antitumor agent" XP002479404 extrait de STN Database accession no. 2003:991890 & EG 21 566 A (EGYPT) 31 décembre 2001 (2001-12-31)
- KAROLAK-WOJCIECHOWSKA, JANINA ET AL: "Structure and activity studies of glycine receptor ligands. Part 8. Arylidene-imidazoline-4-one amino acids" JOURNAL OF MOLECULAR STRUCTURE, vol. 649, no. 1-2, 2003, pages 25-36, XP002479387
- DATABASE CA [Online] CHEMICAL ABSTRACTS SERVICE, COLUMBUS, OHIO, US; CHAZEAU, V. ET AL: "Study of 5-arylidene-2-thiohydantoins with potential immunomodulating and anticancer activities" XP002479405 extrait de STN Database accession no. 1993:124447 & EUROPEAN JOURNAL OF MEDICINAL CHEMISTRY , 27(6), 615-25 CODEN: EJMCA5; ISSN: 0223-5234, 1992,
- DATABASE CA [Online] CHEMICAL ABSTRACTS SERVICE, COLUMBUS, OHIO, US; WATANABE, KINZO ET AL: "A new bioactive triene aldehyde from the marine sponge Leucetta microraphis" XP002479406 extrait de STN Database accession no. 2000:3593 & JOURNAL OF NATURAL PRODUCTS , 63(2), 258-260 CODEN: JNPRDF; ISSN: 0163-3864, 2000,
- DATABASE CA [Online] CHEMICAL ABSTRACTS SERVICE, COLUMBUS, OHIO, US; RENAULT, STEVEN ET AL: "Parallel Solution-Phase Synthesis of 2-Alkylthio-5-arylidene-3,5- dihydro-4H-imidazol-4-one by One-Pot Three-Component Domino Reaction" XP002479407 extrait de STN Database accession no. 2007:1003123 & JOURNAL OF COMBINATORIAL CHEMISTRY , 9(6), 935-942 CODEN: JCCHFF; ISSN: 1520-4766, 2007,
- DATABASE CA [Online] CHEMICAL ABSTRACTS SERVICE, COLUMBUS, OHIO, US; CHEROUVRIER, JEAN RENE ET AL: "A practical and stereoselective route to 5-ylidene-3,5- dihydroimidazol-4-one derivatives using solvent-free conditions under focused microwave irradiations" XP002479408 extrait de STN Database accession no. 2005:618472 & INTERNATIONAL ELECTRONIC CONFERENCES ON SYNTHETIC ORGANIC CHEMISTRY, 5TH, 6TH, SEPT. 1-30, 2001 AND 2002 [AND] 7TH, 8TH, NOV. 1-30, 2003 AND 2004 , 1905-1911. EDITOR(S): SEIJAS, JULIO A. PUBLISHER: MOLECULAR DIVERSITY PRESERVATION INTERNATIONAL, BAS, 2004,
- DATABASE CA [Online] CHEMICAL ABSTRACTS SERVICE, COLUMBUS, OHIO, US; CHEROUVRIER, JEAN-RENE ET AL: "Microwave-mediated solventless synthesis of new derivatives of marine alkaloid Leucettamine B" XP002479409 extrait de STN Database accession no. 2002:303968 & TETRAHEDRON LETTERS , 43(19), 3581-3584 CODEN: TELEAY; ISSN: 0040-4039, 2002,
- DATABASE CA [Online] CHEMICAL ABSTRACTS SERVICE, COLUMBUS, OHIO, US; ROUE, NATHALIE ET AL: "Synthesis of the marine alkaloid leucettamine B" XP002479410 extrait de STN Database accession no. 1999:798095 & TETRAHEDRON , 55(51), 14729-14738 CODEN: TETRAB; ISSN: 0040-4020, 1999,
- DATABASE CA [Online] CHEMICAL ABSTRACTS SERVICE, COLUMBUS, OHIO, US; SHAFI, P. M. ET AL: "A new synthetic route to 4-arylidene-2-phenyl-2-imidazolin-5-ones" XP002479411 extrait de STN Database accession no. 1999:467108 & INDIAN JOURNAL OF CHEMISTRY, SECTION B: ORGANIC CHEMISTRY INCLUDING MEDICINAL CHEMISTRY , 38B(3), 378-379 CODEN: IJSBDB; ISSN: 0376-4699, 1999,
- DATABASE CA [Online] CHEMICAL ABSTRACTS SERVICE, COLUMBUS, OHIO, US; SOBHA, T. D. ET AL: "Synthesis of acylamino acid amides and acylamino acids by the simultaneous reduction and hydrolysis of imidazolinones" XP002479412 extrait de STN Database accession no. 1998:658114 & ASIAN JOURNAL OF CHEMISTRY , 10(4), 1059-1060 CODEN: AJCHEW; ISSN: 0970-7077, 1998,
- DAVIS R A ET AL: "The isolation and synthesis of polyandrocarpamines A and B. Two new 2-aminoimidazolone compounds from the Fijian ascidian, Polyandrocarpa sp", TETRAHEDRON, ELSEVIER SCIENCE PUBLISHERS, AMSTERDAM, NL, vol. 58, no. 16, 15 April 2002 (2002-04-15), pages 3263-3269, XP004347826, ISSN: 0040-4020, DOI: 10.1016/S0040-4020(02)00228-4

## Description

L'invention a pour objet des dérivés d'imidazolones. Elle décrit également un procédé pour leur préparation.

Elle concerne en outre les applications biologiques de ces dérivés comme inhibiteurs de kinases, en particulier pour le traitement de maladies neurodégénératives, (notamment la maladie d'Alzheimer,la maladie de Pick, la trisomie 21).

La grande majorité des pathologies humaines implique des anomalies de phosphorylation souvent associées à des anomalies de régulation de certaines protéines kinases.

La recherche d'inhibiteurs efficaces de ces kinases est ainsi très active depuis ces dernières années (WO 2007/054508; Wo 2006/106046; WO 2006/040052). En s'appuyant sur leur longue expérience concernant les kinases, CDKs, GSK-3 et CK1, les inventeurs se sont intéressés à l'élaboration d'inhibiteurs sélectifs de la kinase DYRK1A (Dual-specificity Tyrosine-phosphorylation-Regulated Kinase 1 A).

Il s'agit d'une enzyme qui s'autophosphoryle sur sa Tyrosine 321 (ce qui conduit à son activation) et qui phosphoryle des résidus Sérine et Thréonine.

Le gène de la protéine kinase DYRK1A est localisé dans une région bien spécifique du chromosome 21, la «Down syndrome critical region», qui couvre une vingtaine de gènes responsables du phénotype trisomique. De nombreux arguments soutiennent l'hypothèse d'une contribution essentielle de la surexpression, même modeste (X 1.5), de DYRK1A dans le développement anormal du cerveau observé au cours de la trisomie 21. Par ailleurs DYRK1A semble également très impliqué dans la maladie d'Alzheimer (qui apparaît chez les trisomiques 21 de façon systématique et précoce au-delà de la quarantaine) (Kimura R, et al., 2006. The DYRK1A gene, encoded in chromosome 21 Down syndrome critical region, bridges between beta-amyloid production and tau phosphorylation in Alzheimer disease. Hum Mol Genet. 16, 15-23; Ferrer I, et al., 2005. Constitutive Dyrk1A is abnormally expressed in Alzheimer disease, Down syndrome, Pick disease, and related transgenic models. Neurobiol Dis. 20,392-400).

Des inhibiteurs de DYRK1A ont été recherchés par criblage virtuel *in silico* sur un modèle structural de DYRK1A basé sur la structure cristalline de GSK-3 (Kim et al., Bioorg.Med.Chem.Lett.,2006 Jul.15 ; 16 (14) :3712-6). Dans cette approche, sur les 182 composés sélectionnés, seules 11 molécules ont montré une activité inhibitrice avec une IC₅₀ variant de 2,5 à 50 µM.

Les travaux des inventeurs pour la recherche, l'optimisation et la caractérisation d'inhibiteurs pharmacologiques de la kinase DYRK1A les ont amené à découvrir que des dérivés d'imidazolones correspondant à des dérivés ou des analogues de la leucettamine B constituaient à cet égard des inhibiteurs pharmacologiques puissants et sélectifs de la kinase DYRK1A. Ci-après le terme "composés" sera également utilisé pour désigner globalement ces dérivés et analogues.

La leucettamine B est un alcaloïde marin extrait de l'éponge *Leucetta microraphis* de formule A

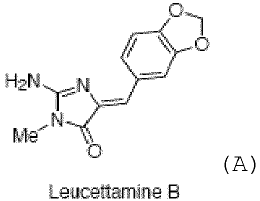

Le développement des recherches a conduit les inventeurs à la mise au point de voies de synthèse permettant d'obtenir une famille de composés dotés de propriétés inhibitrices de grand intérêt vis-à-vis de DYRK1A, avec des IC₅₀ inférieures pour la plupart à 50 µM et même à 10 µM, voire à 1 µM.

L'invention vise donc l'utilisation comme médicaments de dérivés d'imidazolones, constituant des analogues et dérivés de leucettamine B.

Elle décrit également un procédé de préparation de ces composés.

L'invention porte en outre sur les composés qui correspondent à de nouveaux dérivés d'imidazolones et sur leurs applications comme principes actifs de médicaments.

L'invention décrit ainsi l'utilisation, pour fabriquer des médicaments pour le traitement de maladies neurodégénératives, de dérivés d'imidazolone, répondant à la formule (I) 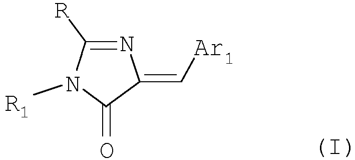 dans laquelle :
- R₁ représente H, un radical alkyle en C1 à C5 linéaire ou ramifié éventuellement substitué ; un groupe aryle, ou un groupe hétérocyclique à 5 ou 6 éléments, le groupe aryle et le groupe hétérocyclique comportant éventuellement un ou plusieurs substituants identiques ou différents, occupant des positions quelconques;
- Ar₁ représente un groupe aryle avec éventuellement un ou plusieurs substituants, deux substituants adjacents pouvant former un cycle à 5 ou 6 éléments, ce cycle étant le cas échéant substitué ; ou un hétérocycle aromatique avec éventuellement un ou plusieurs substituants et/ou condensé avec un cycle aromatique à 5 ou 6 éléments, l'hétéroatome étant choisi parmi N, S et O ;
- R représente R₂-S-, R₃-HN-, R₄COHN ou Ar₂, avec
   - R₂ = un radical alkyle en C1-C5, linéaire, ramifié ou cyclique ;un radical vinyle ou vinyl-alkyle en C1-C5, un radical nitrile ou nitrile-alkyle en C1-C5, un radical aryle, benzyle, lesdits radicaux étant éventuellement substitués sur un ou plusieurs atomes de carbone par un ou plusieurs groupes, identiques ou différents, occupant des positions quelconques, deux substituants adjacents pouvant former un cycle à 5 ou 6 éléments, ce cycle étant le cas échéant substitué,
   - R₃ = les significations données ci-dessus et peut en outre représenter H ;
   - Ar₂ représentant un radical aryle substitué ou non, deux substituants adjacents pouvant former un cycle à 5 ou 6 éléments, ce cycle étant éventuellement substitué.

L'invention décrit aussi les formes racémiques des dérivés ci-dessus ainsi que leurs formes énantiomères prises individuellement.

Comme illustré par les exemples, les dérivés ci-dessus constituent plus spécialement des inhibiteurs sélectifs de la kinase DYRK1A avec des IC₅₀ inférieures à 5 µM, voire inférieures à 1 µM, des dérivés particulièrement avantageux ayant des IC₅₀ inférieures à 0,1 µM.

Dans la formule (I) ci-dessus, «aryle» représente phényle ou naphtyle et «hétérocycle» un cycle à 5 ou 6 éléments avec comme hétéroatome(s) N, O et/ou S. Les substituants de R₁, Ar₁, Ar₂ et R sont choisis parmi : OH, OZ, COH, COZ, COOH, COOZ, NH₂, NHalc., N(alc.)₂, NHCOOH, NHCOOZ, Z représentant un radical alkyle linéaire ou ramifié en C1-C5, aryle, benzyle, aryle ou benzyle substitué, benzodioxolyle, un ou plusieurs halogène et/ou un groupe CCl₃, et alc. représentant un radical alkyle en C1-C3.

L'invention décrit plus spécialement, pour l'utilisation comme médicaments, des dérivés d'imidazolones présentant une IC₅₀ inférieure à 5 µM et répondant à la formule I ci-dessus dans laquelle :
- R₁ représente un radical alkyle en C1-C3 ou un atome d'hydrogène, et/ou un radical aryle
- Ar₁ est choisi parmi 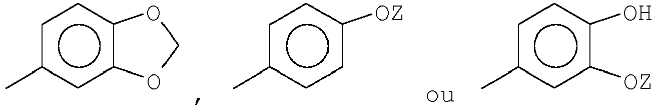
- R représente
   - un groupe R₂-S-, R₂ étant alors choisi parmi les radicaux de type T₁- (CH₂)ₙ, avec n = 0, 1, 2 ou 3 et T₁ représente un radical méthyle, vinyle, alkyle, alkynyle, nitrile, cycloalkyle en C3 ou C4, Z-O, Z-CO, avec Z = alkyle en C1-C3, ou hal, hal représentant F, Cl, Br ou I ou un groupe CCl₃,
      ou
   - un groupe R₃-NH-, R₃ étant alors choisi parmi les radicaux de type T₂- (CH₂)ₙ, avec n = 0, 1 ou 2, et T₂ représente un radical méthyle, vinyle, ZO, ZO-CONH-, - CH-(OZ)₂, ZCO, avec Z = H ou alkyl en C1-C4 linéaire ou ramifié, NH₂, cycloalkyle en C3, aryle, aryle substitué, ou R₂ = H,
      ou
   - un groupe R₄-CONH-, R₄ étant alors un radical alkyle branché en C3-C5,
      ou
   - R = Ar₂, Ar₂ étant choisi parmi un radical phényle, phényle substitué ou benzodioxolyle.

De manière préférée, l'invention décrit, pour l'utilisation comme médicaments, des dérivés d'imidazolones présentant une IC₅₀ inférieure à 1 µM et répondant à la formule I dans laquelle
- R₁ représente H ou CH₃
- Ar₁ représente le radical 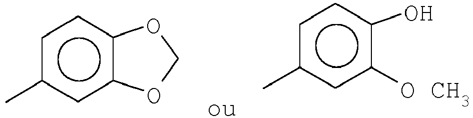
- R₂ représente
   - un groupe R₂-S-, R₂ étant alors choisi parmi les radicaux de type T₁- (CH₂)ₙ, avec T₁ = un radical méthyle, alkynyle, nitrile, hal, CH₃O, cyclopropyle ou cyclobutyle, n = 0, 1, 2 ou 3, « hal » représentant un atome d'halogène ou un groupe CCl₃,
      ou
   - un groupe R₃-HN-, R₃ étant alors choisi parmi les radicaux de type T₂- (CH₂)ₙ, avec T₂ = alkyle en C3, OH, cyclopropyle, n = 0, 1 ou 2, phényle, phényle substitué par OH, OCH₃, COOH et OH, CH₂OH C (CH₃, OH), CH₂-CH₂OH, CH₂-COOH, benzodioxolyle ou R₃ = H,
      ou
   - un groupe Ar₂ choisi parmi un groupe parahydroxyphényle ou benzodioxolyle.

Dans un groupe préféré de dérivés d'imidazolone de formule (I),
- R représente R²S, R³HN, ou Ar² ;
- R¹ représente H ou un radical alkyle en C1-C5, linéaire ou ramifié ;
- R² représente H ou un radical alkyle en C1-C5, linéaire ou ramifié, le cas échéant substitué par un ou plusieurs radicaux OH, alcoxy en C1-C5, (CH₂)ₙ -OH ou (CH₂)ₙ -COOH ; ou représente un radical cyclique, le cas échéant du type -(CH₂)ₙ- cycloalkyle, le radical cycloalkyle ayant 3 à 5 éléments et n=1-5, le radical cyclique étant le cas échéant substitué par un alkyle en C1-C5 ; ou représente un radical C1-C5 alkylène - nitrile ; C1-C5 alkylène-vinyle ; C1-C5 alcynyle ;
- R³ représente un radical alkyle en C1-C5, linéaire ou ramifié, le cas échéant substitué par un ou plusieurs radicaux alcoxy en C1-C5, OH, ou COOH ; ou représente un radical cyclique, le cas échéant du type - (CH₂)ₙ-cycloalkyle ; ou représente un radical phényle le cas échéant substitué par un ou plusieurs - OH, (CH₂)ₙ -OH ; alcoxy ou COOH ; ou un radical benzodioxolyle ; ou représente un radical cyclique, le cas échéant du type - (CH₂)ₙ- cycloalkyle, le radical cycloalkyle ayant 3 à 5 éléments et n=1-5 ; NH₂
- Ar¹ représente un radical benzodioxolyle
- Ar² représente un radical benzodioxolyle ou phényle, ce dernier étant le cas échéant substitué par un ou plusieurs - OH, alcoxy.

Dans un autre groupe préféré de dérivés d'imidazolone de formule (I),
- R représente R²S, R³HN, ou Ar² ;
- R¹ représente H ou un radical alkyle en C1-C5, linéaire ou ramifié ;
- R² représente un radical alkyle en C1-C5, linéaire ou ramifié, le cas échéant substitué par un ou plusieurs radicaux OH, alcoxy en C1-C5, (CH₂)ₙ -OH ; ou représente un radical cyclique, le cas échéant du type -(CH₂)ₙ-cycloalkyle, le radical cycloalkyle ayant 3 à 5 éléments et n=1-5 ; ou représente un radical C1-C5 alkylène - nitrile ; C1-C5 alkylène-vinyle ;
- R³ représente un radical phényle le cas échéant substitué par un ou plusieurs - OH, (CH₂)ₙ-OH ; alcoxy ; ou un radical benzodioxolyle ; ou représente un radical cyclique, le cas échéant du type -(CH₂)ₙ- cycloalkyle, le radical cycloalkyle ayant 3 à 5 éléments et n=1-5 ; NH₂
- Ar¹ représente un radical benzodioxolyle
- Ar² représente un radical benzodioxolyle ou phényle, ce dernier étant le cas échéant substitué par un ou plusieurs - OH.

De tels dérivés d'imidazolones peuvent être choisis parmi les composés suivants dans lesquels, dans la formule (I),
R= R₂S
   R₂ = CH₂C≡CH ; R₁ = Me ; Ar₁ = 1,3-benzodioxol-5-yl
   R₂ = CH₂C≡N ; R₁ = Me ; Ar₁ = 1,3-benzodioxol-5-yl
   R₂ = CH₂CH₂Cl ; R₁ = Me ; Ar₁ = 1,3-benzodioxol-5-yl
   R₂ = CH₃ ; R₁ = H ; Ar₁ = 1,3-benzodioxol-5-yl
   R₂ = CH₂CH₃ ; R₁ = H ; Ar₁ = 1,3-benzodioxol-5-yl
   R₂ = CH₂CH₂CH₃ ; R₁ = H ; Ar₁ = 1,3-benzodioxol-5-yl
   R₂ = CH(CH₃)₂ ; R₁ = H ; Ar₁ = 1,3-benzodioxol-5-yl
   R₂ = CH₂C≡N ; R₁ = H ; Ar₁ = 1,3-benzodioxol-5-yl
   R₂ = CH₂ (CH₂)₂CH₃ ; R₁ = H ; Ar₁ = 1,3-benzodioxol-5-yl
   R₂ = CH₂CH₂OCH₃ ; R₁ = H ; Ar₁ = 1,3-benzodioxol-5-yl
   R₂ = CH₂T₁ avec T₁= cyclopropyl ; R₁ = H ; Ar₁ = 1,3-benzodioxol-5-yl
   R₂ = CH₂T₁ avec T₁= cyclobutyl ; R₁ = H ; Ar₁ = 1,3-benzodioxol-5-yl
R=R₃NH
   R₃ = CH₂CH₃ ; R₁ = Me ; Ar₁ = 1,3-benzodioxol-5-yl
   R₃ = CH₂CH₂OH R₁ = Me ; Ar₁ = 1,3-benzodioxol-5-yl
   R₃ = CH₂T₁ avec T₁= cyclopropyl ; R₁ = Me ; Ar₁ = 1,3-benzodioxol-5-yl
   R₃ = CH₂CH₃ ; R₁ = Me ; Ar₁ = 1,3-benzodioxol-5-yl
   R₃ = o-HO-C₆H₄ ; R₁ = Me ; Ar₁ = 1,3-benzodioxol-5-yl
   R₃ = C₆H₅ ; R₁ = Me ; Ar₁ = 1,3-benzodioxol-5-yl
   R₃ = p-HO-C₆H₄ ; R₁ = Me ; Ar₁ = 1,3-benzodioxol-5-yl
   R₃ = p-HO-m-HO₂C-C₆H₃ ; R₁ = Me ; Ar₁ = 1,3-benzodioxol-5-yl
   R₃ = p-m-OCH₂O-C₆H₃ ; R₁ = Me ; Ar₁ = 1,3-benzodioxol-5-yl
   R₃ = p-CH₃-C₆H₄ ; R₁ = Me ; Ar₁ = 1,3-benzodioxol-5-yl
   R₃ = HOCH₂CHOHCH₂ ; R₁ = Me ; Ar₁ = 1, 3-benzodioxol-5-yl
   R₃ = p-m-OCH₂CH₂O-C₆H₃ ; R₁ = Me ; Ar₁ = 1,3-benzodioxol-5-yl
   R₃ = p-CH₃O-C₆H₄ ; R₁ = Me ; Ar₁ = 1,3-benzodioxol-5-yl
   R₃ = m-HOCH_{Z}-C₆H₄ ; R₁ = Me ; Ar₁ = 1,3-benzodioxol-5-yl
   R₃ = m-HOCH (CH₃) -C₆H₄ ; R₁ = Me ; Ar₁ = 1,3-benzodioxol-5-yl
   R₃ = p-HOCH₂CH₂-C₆H₄ ; R₁ = Me ; Ar₁ = 1,3-benzodioxol-5-yl
   R₃ = p-HO₂CCH₂O-C₆H₄ ; R₁ = Me ; Ar₁ = 1,3-benzodioxol-5-yl
   R₃ = CH₂CH₂CH₃ ; R₁ = H ; Ar₁ = 1,3-benzodioxol-5-yl
   R₃ = CH₂T₁ avec T₁= cyclopropyl ; R₁ = H ; Ar₁ = 1,3-benzodioxol-5-yl
   R₃ = C₆H₅ ; R₁ = H ; Ar₁ = 1,3-benzodioxol-5-yl
   R₃ = p-HO-C₆H₄ ; R₁ = H ; Ar₁ = 1,3-benzodioxol-5-yl
   R₃ = H ; R₁ = H ; Ar₁ = p-HO-m-MeO-C₆H₃
R= Ar₂
   Ar₂ = p-HO-C₆H₄ ; R₁ = Me ; Ar₁ = 1,3-benzodioxol-5-yl
   Ar₂ = p-m-OCH₂O-C₆H₃ ; R₁ = Me ; Ar₁ = 1,3-benzodioxol-5-yl L'invention vise des dérivés d'imidazolones de formule (I) 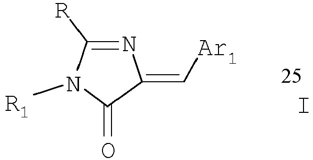
pour leur utilisation dans le traitement de maladies neurodégénératives, de la maladie de Pick et de la trisomie 21, choisis parmi les dérivés de formule (I) dans lesquels
**Rr=R₂S**
   R₂= CH₂C=CH; R₁ = CH₃; Ar₁ = 1,3-benzodioxol-5-yl,
   R₂= CH₂C=N; R₁ = CH₃; Ar₁ = 1,3-benzodioxol-5-yl,
   R₂= CH₂CH₂Cl; R₁ = Me; Ar₁ = 1,3-benzodioxol-5-yl,
   R₂= CH₃; R₁ = H; Ar₁ = 1,3-benzodioxol-5-yl,
   R₂= CH₂CH₃; R₁ = H; Ar₁ = 1,3-benzodioxol-5-yl,
   R₂= CH₂CH₂CH₃; R₁ = H; Ar₁ = 1,3-benzodioxol-5-yl,
   R₂= CH(CH₃)₂; R₁ = H; Ar₁ = 1,3-benzodioxol-5-yl,
   R₂= CH₂C=N; R₁ = H; Ar₁ = 1,3-benzodioxol-5-yl,
   R₂= CH₂CH₂CH₂CH₃; R₁ = H; Ar₁ = 1,3-benzodioxol-5-yl,
   R₂= CH₂CH₂OCH₃; R₁ = H; Ar₁ = 1,3-benzodioxol-5-yl,
   R₂= CH₂T1 avec T1 = cyclopropyl; R₁ = H; Ar₁ = 1,3-benzodioxol-5-yl,
   R₂= CH₂T1 avec T1= cyclobutyl; R₁ = H; Ar₁ = 1,3-benzodioxol-5-yl,
**R=R₃NH**
   R₃= CH₂CH₃; R₁ = CH₃; Ar₁ = 1,3-benzodioxol-5-yl,
   R₃= CH₂CH₂OH; R₁ = CH₃; Ar₁ = 1,3-benzodioxol-5-yl,
   R₃= CH₂CH (OH) (CH₂OH); R₁ = CH₃; Ar₁ = 1, 3-benzodioxol-5-yl,
   R₃= CH₂T1 avec T1 - cyclopropyl; R₁ = CH₃; Ar₁ = 1,3-benzodioxol-5-yl,
   R₃= o-OH-C₆H₄; R₁ = CH₃; Ar₁ = 1,3-benzodioxol-5-yl,
   R₃= p-OH-C₆H₄; R₁ = CH₃; Ar₁ = 1,3-benzodioxol-5-yl,
   R₃= p-OH, m-CO₂H-C₆H₄; R₁ = CH₃; Ar₁ = 1,3-benzodioxol-5-yl,
   R₃= 1,3-benzodioxol-5-yl; R₁ = CH₃; Ar₁ = 1,3-benzodioxol-5-yl,
   R₃= p-m-OCH₂CH₂O-C₆H₃; R₁ = CH₃; Ar₁ = 1,3-benzodioxol-5-yl,
   R₃= p-OCH₃-C₆H₄; R₁ = CH₃; Ar₁ = 1,3-benzodioxol-5-yl,
   R₃= m-CH₂OH-C₆H₄; R₁ = CH₃; Ar₁ = 1,3-benzodioxol-5-yl,
   R₃= m-HOCH (CH₃)-C₆H₄; R₁ = CH₃; Ar₁ = 1,3-benzodioxol-5-yl,
   R₃= C₆H₅; R₁ = CH₃; Ar₁ = 1,3-benzodioxol-5-yl,
   R₃= p-CH₂COOH-C₆H₄; R₁ = CH₃; Ar₁ = 1,3-benzodioxol-5-yl,
   R₃= CH₂CH₂CH₃; R₁ = H; Ar₁ = 1,3-benzodioxol-5-yl,
   R₃= -C₆H₅; R₁ = H; Ar₁ = 1,3-benzodioxol-5-yl,
   R₃= -p-OH-C₆H₄; R₁ = H; Ar₁ = 1,3-benzodioxol-5-yl,
   R₃= CH₂T1 avec T1 = cyclopropyl; R₁ = H; Ar₁ = 1,3-benzodioxol-5-yl,
   R₃= H; R₁ = H; Ar₁ = p-OH, m-OCH₃-C₆H₃,
**R=Ar₂**
   Ar₂= -p-OH-C₆H₄; R₁ = CH₃; Ar₁ = 1,3-benzodioxol-5-yl,
   Ar₂= 1,3-benzodioxol-5-yl; R₁ = CH₃; Ar₁ = 1,3-benzodioxol-5-yl.

Selon un autre aspect, l'invention vise de nouveaux dérivés d'imidazolones.

Plus particulièrement, l'invention vise des dérivés d'imidazolones de formule (I) 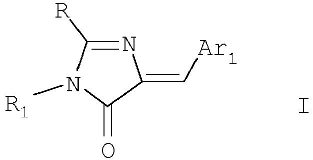 choisis parmi les dérivés dans lesquels
**R=R₂S**
   R₂= CH₂C≡N; R₁ = CH₃; Ar₁ = 1,3-benzodioxol-5-yl,
   R₂= CH₂CH₂Cl; R₁ = Me; Ar₁ = 1,3-benzodioxol-5-yl,
   R₂= CH₃; R₁ = H; Ar₁ = 1,3-benzodioxol-5-yl,
   R₂= CH₂CH₃; R₁ = H; Ar₁ = 1,3-benzodioxol-5-yl, R₂= CH₂CH₂CH₃; R₁ = H; Ar₁ = 1,3-benzodioxol-5-yl,
   R₂= CH(CH₃)₂; R₁ = H; Ar₁ = 1,3-benzodioxol-5-yl,
   R₂= CH₂C≡N; R₁ = H; Ar₁ = 1,3-benzodioxol-5-yl,
   R₂= CH₂CH₂CH₂CH₃; R₁ = H; Ar₁ = 1,3-benzodioxol-5-yl,
   R₂= CH₂CH₂OCH₃; R₁ = H; Ar₁ = 1,3-benzodioxol-5-yl,
   R₂= CH₂T1 avec T1 = cyclopropyl; R₁ = H; Ar₁ = 1,3-benzodioxol-5-yl,
   R₂= CH₂T1 avec T1= cyclobutyl; R₁ = H; Ar₁ = 1, 3-benzodioxol-5-yl,
**R=R₃NH**
   R₃= CH₂CH₃; R₁ = CH₃; Ar₁ = 1,3-benzodioxol-5-yl,
   R₃= CH₂CH₂OH; R₁ = CH₃; Ar₁ = 1,3-benzodioxol-5-yl,
   R₃= CH₂CH (OH) (CH₂OH) ; R₁ = CH₃; Ar₁ = 1, 3-benzodioxol-5-yl,
   R₃= CH₂T1 avec T1 = cyclopropyl; R₁ = CH₃; Ar₁ = 1,3-benzodioxol-5-yl,
   R₃= o-OH-C₆H₄; R₁ = CH₃; Ar₁ = 1,3-benzodioxol-5-yl,
   R₃= p-OH-C₆H₄; R₁ = CH₃; Ar₁ = 1,3-benzodioxol-5-yl,
   R₃= p-OH, m-CO₂H-C₆H₄; R₁ = CH₃; Ar₁ = 1,3-benzodioxol-5-yl,
   R₃= 1,3-benzodioxol-5-yl; R₁ = CH₃; Ar₁ = 1,3-benzodioxol-5-yl,
   R₃= p-m-OCH₂CH₂O-C₆H₃; R₁ = CH₃; Ar₁ = 1,3-benzodioxol-5-yl,
   R₃= p-OCH₃-C₆H₄; R₁ = CH₃; Ar₁ = 1,3-benzodioxol-5-yl,
   R₃= m-CH₂OH-C₆H₄; R₁ = CH₃; Ar₁ = 1,3-benzodioxol-5-yl,
   R₃= m-HOCH(CH₃)-C₆H₄; R₁ = CH₃; Ar₁ = 1, 3-benzodioxol-5-yl,
   R₃= C₆H₅; R₁ = CH₃; Ar₁ = 1,3-benzodioxol-5-yl,
   R₃= p-CH₂COOH-C₆H₄; R₁ = CH₃; Ar₁ = 1,3-benzodioxol-5-yl,
   R₃= CH₂CH₂CH₃; R₁ = H; Ar₁ = 1,3-benzodioxol-5-yl,
   R₃= -C₆H₅; R₁ = H; Ar₁ = 1,3-benzodioxol-5-yl,
   R₃= -p-OH-C₆H₄; R₁ = H; Ar₁ = 1,3-benzodioxol-5-yl,
   R₃= CH₂T1 avec T1 = cyclopropyl; R₁ = H; Ar₁ = 1,3-benzodioxol-5-yl,
**R=Ar₂**
   Ar₂= -p-OH-C₆H₄; R₁ = CH₃; Ar₁ = 1,3-benzodioxol-5-yl,
   Ar₂= 1,3-benzodioxol-5-yl; R₁ = CH₃; Ar₁ = 1,3-benzodioxol-5-yl

Ces nouveaux dérivés, dans leur utilisation comme médicaments, font également partie de l'invention.

L'invention vise donc des compositions pharmaceutiques comprenant une quantité thérapeutiquement efficace des dérivés de formule (I) définis ci-dessus.

Comme le montrent les valeurs de IC₅₀ rapportées dans les exemples, les composés définis ci-dessus constituent des inhibiteurs puissants de la kinase DYRK1A et à ce titre sont utiles à la fois en tant qu'outils pharmacologiques pour la recherche fondamentale et en tant qu'agents thérapeutiques pour le traitement de maladies neurodégénératives, en particulier de la maladie d'Alzheimer et autres taupathies, de la maladie de Pick et de la trisomie 21.

Les dérivés de formule I ou les nouveaux dérivés selon l'invention constituent en effet des outils permettant d'étudier les fonctions de DYRK1A dans divers modèles cellulaires et les conséquences de son expression et d'une activité anormale. Ils constituent des principes actifs de médicaments pour contrer les effets de la surexpression/ activation anormale de DYRK1A dans les pathologies ci-dessus.

Lors de l'élaboration des médicaments, les principes actifs, utilisés en quantités thérapeutiquement efficaces, sont mélangés avec les véhicules pharmaceutiquement acceptables pour le mode d'administration choisi.

Ainsi pour une administration par voie orale, les médicaments sont préparés sous forme de gélules, comprimés, dragées, capsules, pilules, gouttes et analogues. De tels médicaments peuvent renfermer de 1 à 100 mg de principe actif par unité.

Pour l'administration par voie injectable (intraveineuse, sous-cutanée, intramusculaire), les médicaments se présentent sous forme de solutions stériles ou stérilisables. Les doses par unité de prise peuvent varier de 1 à 50 mg de principe actif. La posologie quotidienne est choisie de manière à obtenir une concentration finale d'au plus 100 µM en analogue ou dérivé d'imidazolone dans le sang du patient traité.

La présente invention décrit également un procédé de synthèse des dérivés d'imidazolone de formule I définis ci-dessus.
Ce procédé est caractérisé en ce qu'il comprend l'utilisation d'un dérivé d'arylidène thiohydantoïne répondant à la formule 3 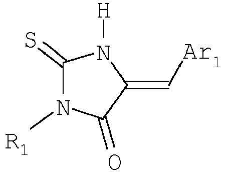 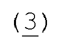 dans laquelle
R₁, R₂, Ar₁ sont tels que définis ci-dessus.

Selon un mode de réalisation visant à préparer les dérivés d'imidazolone de formule I dans laquelle R = R₂S, le procédé comprend la réaction d'un dérivé de thiohydantoïne 3 avec un dérivé halogéné 3' de formule

R₂ X (3')

avec X = Cl, Br ou I
dans des conditions permettant d'obtenir un dérivé d'imidazolone 4, selon le schéma 4 suivant

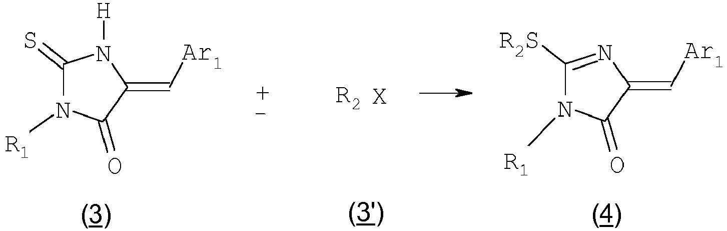

De manière avantageuse, la réaction entre les composés 3 et 3' est réalisée dans un solvant organique, à une température de 70 à 100°C, notamment de 80°C, en présence de carbonate.
Pour obtenir plus particulièrement des dérivés de formule (I) dans lesquels R₂ est un radical aryle, le procédé de l'invention décrit la réaction du dérivé de thiohydantoïne 3 avec un acide arylboronique 7' de formule

Ar₂ B(OH)₂ (7')

dans des conditions permettant de conduire aux dérivés de formule 8, selon le schéma 1' :

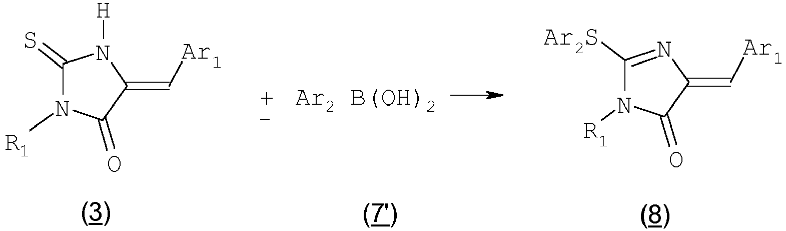

Des conditions satisfaisantes correspondent à une réaction sous micro-onde de la thioxohydantoïne avec l'acide boronique en présence de Cu(AcO)₂, (avec Ac = acétyle) et de phénanthroline dans un solvant organique tel que le dichloroéthane.

Le mélange est irradié de 50 à 100 min, notamment de 60 à 90 min, à 70-90°C, notamment 80°C, avec une puissance maximale de 300 Watts environ.
Selon un mode de réalisation visant à préparer les dérivés d'imidazolone de formule I dans laquelle R = R₃ HN, le procédé de l'invention comprend
- soit la réaction d'un dérivé d'imidazolone tel que défini ci-dessus 4 avec une amine 4' de formule

   R₃-NH₂ (4')

   dans des conditions permettant d'obtenir le dérivé d'imidazolone 5 de formule, selon le schéma 2 suivant : 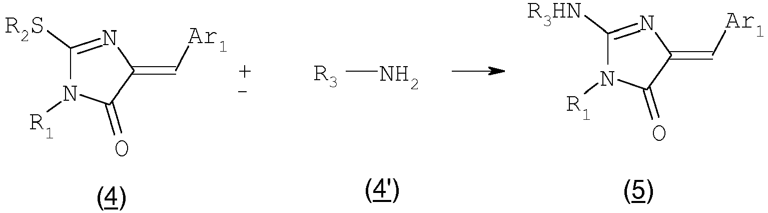
- soit la réaction d'un dérivé d'hydantoïne 3 tel que défini ci-dessus avec une amine 4', selon le schéma 3 suivant ;

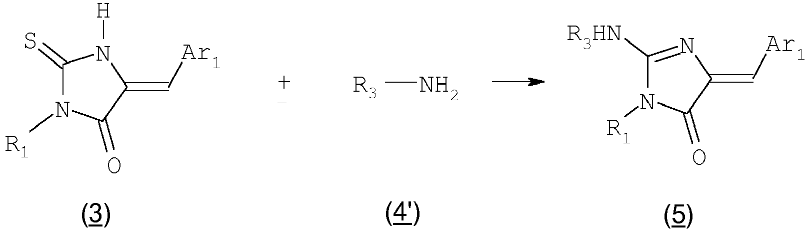

De préférence, la réaction selon le schéma 3 est réalisée au bain d'huile ou sous micro-onde. Dans le mode opératoire au bain d'huile, le mélange réactionnel est chauffé à une température inférieure au point d'ébullition de l'amine. Lorsqu'on opère sous micro-onde, le mélange est avantageusement irradié de 10 à 100 minutes à une température et une puissance appropriées.

La réaction selon le schéma 3 est avantageusement conduite dans un solvant tel que le méthanol en présence d'hydroperoxyde.

Selon un mode de réalisation visant à préparer les dérivés d'imidazolone de formule I dans laquelle R = R₄ COHN, le procédé comprend la réaction d'un dérivé d'imidazolone 5 de formule 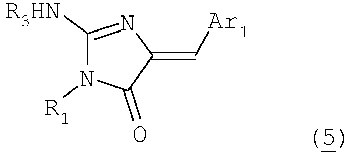 avec un chlorure d'acide 5' de formule

R₄COCl (5')

dans des conditions permettant d'obtenir un dérivé d'imidazolone 6, selon le schéma 4 suivant :

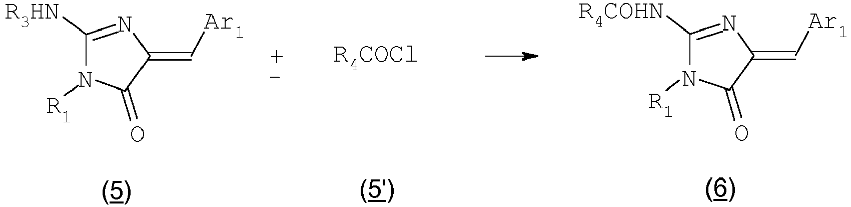

Les substituants dans ces différentes formules sont tels que définis ci-dessus.

Des conditions appropriées pour la réalisation de la réaction entre ces dérivés comprennent l'addition de triéthylamine, puis du chlorure d'acide (5') à une solution dans un solvant organique tel que le THF du dérivé d'imidazolone 5.

Cette réaction est réalisée avantageusement à une température de l'ordre de 20 à 25°C.

Pour préparer les dérivés d'imidazolone de formule I dans laquelle R = Ar₂, le procédé de l'invention comprend la réaction d'un dérivé de thiohydantoïne de formule 3 avec un acide boronique 7'

Ar₂ B(OH)₂ (7')

dans des conditions permettant d'obtenir un dérivé d'imidazolone 7, selon le schéma 5 suivant :

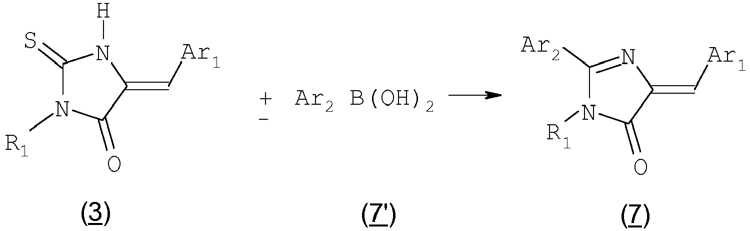

Cette réaction est avantageusement réalisée en présence d'un catalyseur tel que Pd(PPh₃)₄ et de CuTC (Cuivre Thiophène Carboxylate) dans un solvant organique anhydre tel que du THF anhydre.Cette réaction est réalisée avantageusement à une température de l'ordre de 55 à 65°C.

De manière plus particulièrement préférée, le dérivé de thiohydantoïne 3 est obtenu par réaction d'un dérivé de thiohydantoïne de formule 2 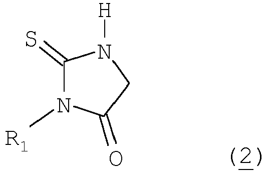 avec un dérivé d'aldimine de formule 2'

Ar₁-CH=N-alk (2')

les substituants étant tels que définis ci-dessus et «alk» représentant un radical alkyle en C3-C5, selon le schéma 6 suivant

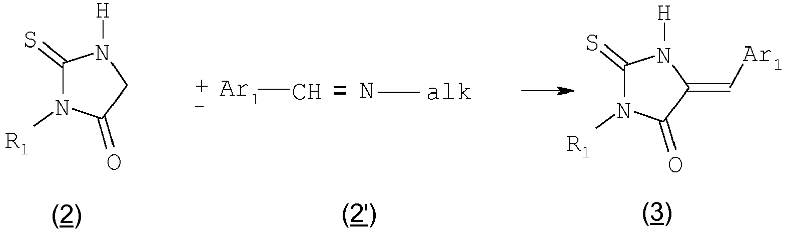

Avantageusement, la réaction est réalisée au bain d'huile ou sous-micro-onde.

Dans le mode opératoire au bain d'huile, les réactifs sont ajoutés dans un solvant organique et le mélange réactionnel porté au reflux. Une huile visqueuse qui cristallise rapidement est récupérée après filtration et purifiée si souhaité.

Parmi les solvants organiques appropriés, on citera l'acétonitrile.

Dans le mode opératoire sous micro-onde, le mélange de thiohydantoïne de formule 2 et d'aldimine de formule 2' est placé dans le réacteur micro-onde, introduit dans un four micro-onde, où le mélange est irradié, puis à la fin de la réaction et retour à la température ambiante, le produit réactionnel est récupéré.

Des conditions appropriées comprennent un traitement d'environ 1 h à 70 - 100°C, notamment de 80°C, avec une puissance maximale de 80 - 100 Watts, plus spécialement de 90 Watts.

L'aldimine 2' est obtenue par exemple à partir d'un aldéhyde 2" Ar₁ - CH = O et de propylamine 2''' CH₃-(CH₂)₂-NH₂. Cette réaction est avantageusement réalisée dans un réacteur micro-onde à une puissance de 300 Watts par exemple à 20-80°C, notamment 25 à 60°C, pendant 2 à 5 minutes, notamment 3 minutes, puis à 60-80°C à puissance réduite de l'ordre de 10 à 30% notamment de 20%.

Le dérivé de thiohydantoïne 2 est de préférence obtenu par réaction de chlorhydrate du glycinate de méthyle 1

CH₃O₂C-CH₂-NH₂, HCl (1)

avec un isothiocyanate 1' de formule

R₁-N=C=S (1')

selon le schéma 7 suivant :

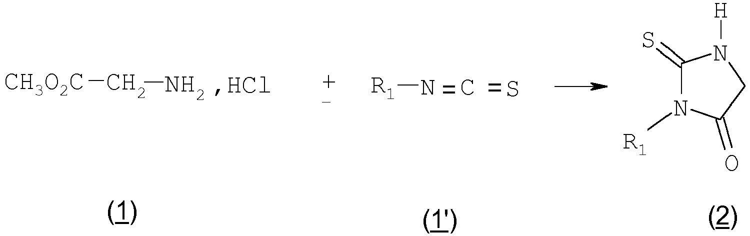

Des conditions de réaction satisfaisantes comprennent la réaction de 1 et 1' en présence de triéthylamine, dans un solvant tel que l'éther à reflux.

D'autres caractéristiques et avantages de l'invention sont donnés dans les exemples qui suivent de mode de réalisation de l'invention portant sur la synthèse des dérivés d'imidazolone selon l'invention.

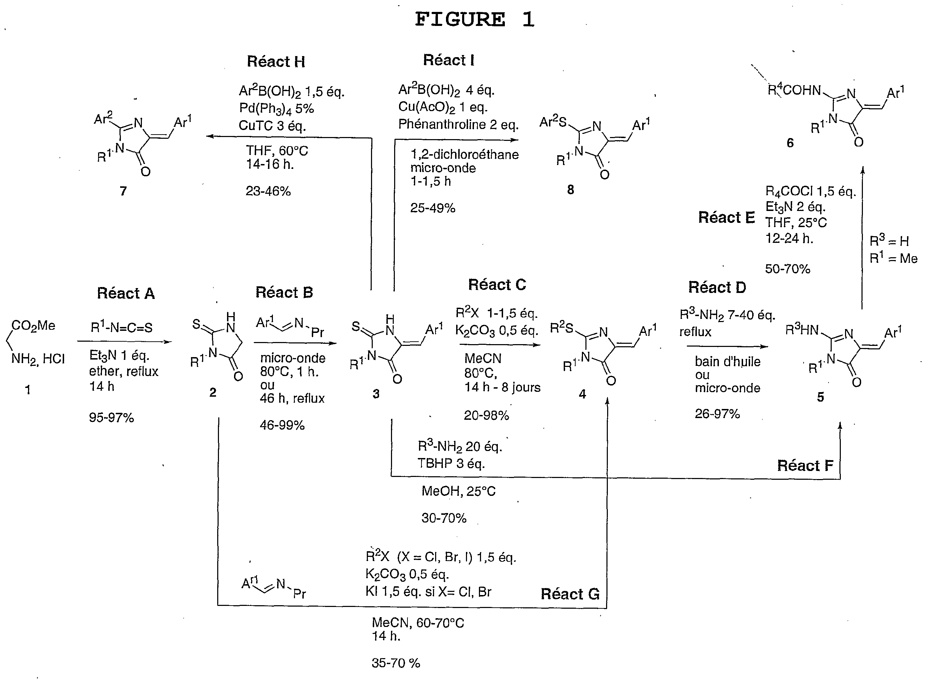

A titre illustratif, les conditions expérimentales des réactions désignées par A à H, résumées dans la figure 1, sont rapportées, dans la partie expérimentale qui suit.

Les valeurs d' IC₅₀ en µM vis-à-vis de DYRK1A de composés selon l'invention sont ensuite données dans le Tableau 2 dans la partie relative au dosage de l'activité kinase de DYRD1A

### Partie expérimentale

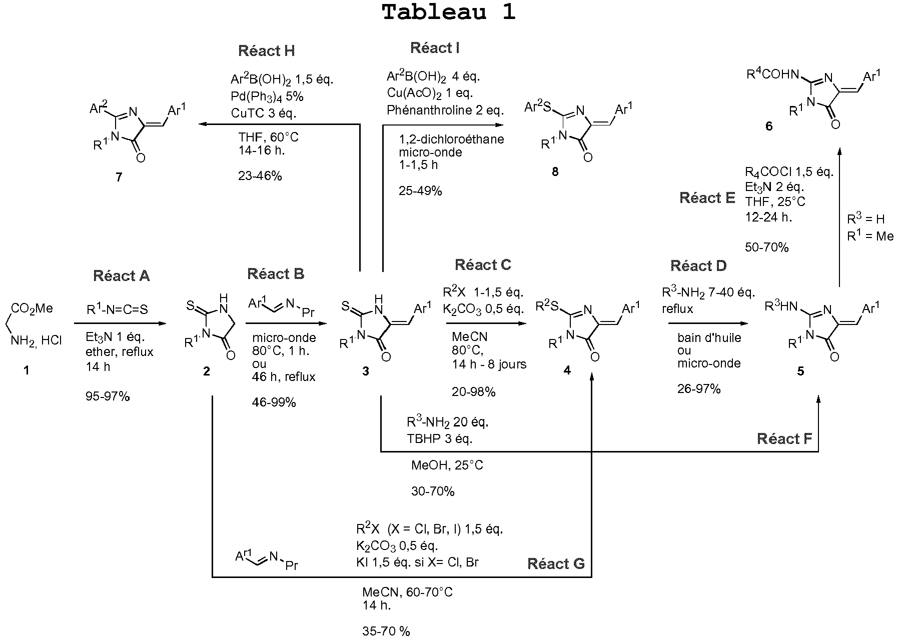

### REACTION A:

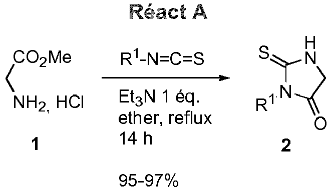

Mode opératoire général: Un mélange constitué de 7 mmoles d'isothiocyanate (R¹N=C=S), 7 mmoles (0,88 g) de chlorhydrate du glycinate de méthyle **1,** 7 mmoles (0,97 mL) de triéthylamine dans 15 mL d'éther est chauffé pendant 14 heures au reflux du solvant sous vive agitation magnétique. Après refroidissement du milieu de réaction à la température ambiante, le solvant est éliminé sous pression réduite à l'évaporateur rotatif. Le chlorhydrate de triéthylamine est éliminé par précipitation dans l'acétate d'éthyle. Après filtration sur verre fritté de porosité N°4, le filtrat est concentré à l'évaporateur rotatif sous pression réduite et on obtient le produit **2** attendu. Celui-ci est utilisé ultérieurement sans purification supplémentaire.

### Exemple de composé 2:

### 3-méthyl-2-thioxo-imidazolidin-4-one (R¹ = Me).

Rdt = 95%, mp = 170-172°C. RMN ¹H (200 MHz, CDCl₃, TMS) δ : 3,27 (s, 3H, NCH₃) ; 4, 11 (s, 2H, -CH₂-); 7,64 (s large, 1H, NH) . RMN ¹³C (75 MHz, CDCl₃, TMS) δ : 27,6 (NCH₃) ; 48,6 (-CH₂-); 171,6 (C=O) ; 185,4 (C=S).

### REACTION B:

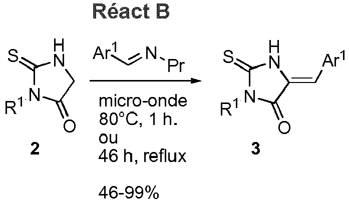

**Mode opératoire général au bain d'huile:** Dans un ballon muni d'un barreau aimanté, sont ajoutés successivement du dichlorométhane (20 mL), 6,9 mmoles de la thiohydantoïne 2, puis 6,9 mmoles de l'aldimine(*) fraîchement distillé. Le mélange réactionnel est ensuite porté au reflux du dichlorométhane et la réaction est suivie par chromatographie sur couche mince de silice 60 F 254 (Merck). Lorsque la réaction est terminée, le milieu réactionnel est refroidi à la température ambiante, puis est séché sur MgSO₄ anhydre. Après filtration sur papier plissé, le solvant du filtrat est éliminé par évaporation sous pression réduite et on obtient une huile visqueuse qui cristallise rapidement à la température ambiante. La purification est réalisée soit par recristallisation dans le pentane soit éventuellement par chromatographie sur gel de silice 60F 254 (Merck) avec un solvant approprié.

**Mode opératoire général sous micro-onde:** Un mélange constitué de 10 mmol de la thiohydantoïne 2 et 10 mmol (1 équivalent) d'aldimine(*) est placé dans un réacteur micro-onde de forme cylindrique (0 = 4 cm). Le réacteur est ensuite introduit dans le four micro-onde Synthewave 402 (marque Prolabo, groupe Merck-Eurolab) muni d'un système d'agitation à pales. Le mélange est irradié pendant 1 heure à 80°C (palier de 3 minutes) avec une puissance maximale de 90 Watts (micro-onde Prolabo). Après retour à la température ambiante, le mélange réactionnel est ensuite concentré à l'évaporateur rotatif. Une solution d'un mélange chloroforme/pentane (1/2) est ajouté au résidu d'évaporation. Après trituration de ce mélange, le solide insoluble est filtré sur verre fritté de porosité N°4 puis séché sous pression réduite.

### Exemple de composé 3:

### (5Z)-5-(1,3-Benzodioxol-5-ylmethylene)-3-methyl-2-thioxoimidazolidin-4-one (Ar¹ = 1,3-benzodioxol-5-yl, R¹ = Me):

Rdt = 87%. Poudre jaune, mp = 253-255°C. RMN ¹H (300 MHz, DMSO-d₆) δ = 3,18 (s, 3H, NCH₃) ; 6,09 (s, 2H, OCH₂O) ; 6,54 (s, 1H, C=C**H**) ; 6,96 (d, 1H, *J* = 8,1 Hz, H-5) ; 7,27 (d, 1H, *J* = 8,1 Hz, H-6) ; 7,45 (s, 1H, H-2) ; 12,22 (sl, 1H, NH). RMN ¹³C (75 MHz, DMSO-d₆) δ= 27,6 (NCH₃) ; 102,1 (OCH₂O) ; 109,1 (C-5) ; 109,8 (C-2) ; 113,7 (C=C**H**) ; 125,1 (**C**=CH) ; 126,9 (C-6) ; 126,9 (C-1) ; 148,4 (C-4) ; 149,0 (C-3) ; 164,6 (C=O) ; 179,0 (C=S). SMHR, *m*/*z* : 262,0409 (calculée pour C₁₂H₁₀N₂O₃S : 262,0412) .

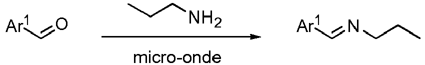

*(*) Mode opératoire général pour la synthèse des aldimines:* 20 mmoles d'aldéhyde et 40 mmoles (3,28 mL) de propylamine sont pésées successivement dans un réacteur en quartz. Ce milieu réactionnel est chauffé dans le réacteur micro-onde Synthewave 402 (P_{max·} = 300 W, marque Prolabo, groupe Merck-Eurolab) selon la programmation suivante (de 25 à 60°C pendant 3 minutes puis à 60°C pendant 30 minutes avec une puissance de 20%). L'excès de propylamine est éliminé à l'évaporateur rotatif sous vide partiel puis le résidu d'évaporation (état solide) est mis en solution dans du dichloromethane (10 mL/gr. de produit); la solution organique est ensuite séchée sur MgSO₄, filtée sur papier filtre. Le filtrat est concentré à l'évaporateur rotatif sous pression réduite.

### Exemple d'aldimine:

### N-[(1,3)-benzodioxol-5-ylméthylène]-N-propylamine (Ar¹ = 1,3-benzodioxol-5-yl):

Rdt = 97%. Poudre de jaune. RMN ¹H (200 MHz, CDCl₃) δ: 0,90 (t, 3H, J = 7,3 Hz, NCH₂CH₂C**H**₃) ; 1,64 (st, 2H, J = 7,2 Hz, NCH₂C**H₂**CH₃) ; 3,47 (t, 2H, J = 6, 9 Hz, NCH₂C**H₂**CH₃) ; 5,90 (s, 2H, OCH₂O) ; 6,71 (d, 1H, J = 7,9 Hz, H-5) ; 7,02 (dd, 1H, J = 1,3; 7,9 Hz, H-6) ; 7,37 (d, 1H, J = 1,4 Hz, H-2) ; 8,10 (s, 1H, N=CH). RMN ¹³C (75 MHz, CDCl₃) δ: 12,2 (CH₃) ; 24,5 (**CH₂**CH₃) ; 63,7 (NCH₂) ; 101,8 (OCH₂O) ; 107,0 (C-3) ; 108,4 (C-6) ; 124,5 (C-2) ; 131,6 (C-1) ; 148,6 (C-5) ; 150,0 (C-4) ; 160,3 (N=CH).

### REACTION C:

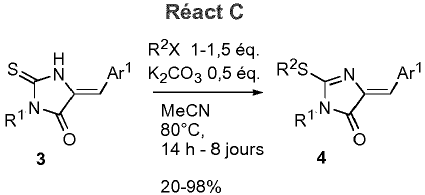

**Mode opératoire général:** Dans un ballon on ajoute successivement la 5-arylidene thiohydantoïne **3** (3,1 mmoles, 1 éq.), 20 mL d'acétonitrile, le dérivé halogéné R²X avec X = Cl, Br, I (3, 1 mmoles, 1 éq.) et 0, 21 g de K₂CO₃ (1,5 mmoles, 0,5 éq.). Le mélange réactionnel est chauffé à 80°C pendant 14 heures sous vive agitation magnétique. Après refroidissement à l'ambiante, l'acétonitrile est éliminé à l'évaporateur rotatif sous pression réduite. Au milieu réactionnel brut, on ajoute 20 mL d'éther. Après filtration sous vide partiel des produits minéraux insolubles sur verre fritté de porosité N°4, le filtrat est séchée sur sulfate de magnésium puis filtrée sur papier plissé. Le solvant du filtrat est éliminé à l'évaporateur rotatif sous pression réduite et on obtient l'imidazolone **4** attendu sous la forme de poudre.

### Exemple de composé 4:

### [(Z)-(4-Benzo[1,3]dioxol-5-ylméthylène-1-méthyl-5-oxo-4,5-dihydro-1H-imidazol-2-ylsulfanyl)]-acétate d'éthyle (Ar¹ = 1,3-benzodioxol-5-yl, R¹ = Me, R² = CH₂CO₂Et) :

Rdt = 92 %. Poudre jaune, mp = 172-174°C. RMN ¹H (300 MHz, CDCl₃) δ : 1,26 (t, 3H, *J* 7,1 Hz, OCH₂CH₃) ; 3,12 (s, 3H, NMe) ; 4,02 (s, 2H, CH₂); 4,23 (q, 2H, *J* 7,1 Hz, OCH₂CH₃) ; 5,96 (s, 2H, OCH₂O); 6,80 (d, 1H, *J* 8,1 Hz); 6,84 (s, 1H, =CH) ; 7,52 (dd, 1H, *J* 8,1; 1,3 Hz) ; 7,96 (d, 1H, J 1, 3 Hz) . RMN ¹³C (75 MHz, CDCl₃) δ: 14,1 (qm, J 128 Hz, OCH₂CH₃); 26,6 (q, *J* 144 Hz, NMe) ; 32,9 (t, *J* 144 Hz, SCH₂) ; 62,3 (tq, J 148; 4,6 Hz, OCH₂); 101,5 (t, J 174 Hz, OCH₂O); 108,4 (d, *J* 165 Hz); 110,8 (dt, J 168; 6,9 Hz); 124,6 (dt, J 156; 4,1 Hz); 128,4 (dt, *J* 162; 6,0 Hz); 128,9 (d, J 7,6 Hz); 136,6 (s); 148,0 (m, =C-O); 149,3 (m, =C-O); 162,0 (m, C-2); 168,0 (m, C=O (CO₂Et)); 169,7 (m, C-4). SMHR, m/z: 348,0791 (calc. pour C₁₆H₁₆N₂O₅S: 348,0780).

### REACTION D:

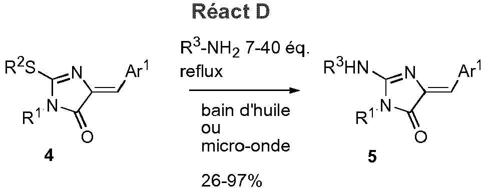

**Mode opératoire sous micro-onde:** Un mélange constitué de (5Z)-5-arylidene-2-alkylthio-3,5-dihydroimidazol-4-one **4** (4 mmoles, 1 éq.) et de 5-20 mmoles d'aminoalcool R³-NH₂ (1,5 à 5 équivalents) est placé dans un réacteur micro-onde de forme cylindrique (0 = 4 cm). Le réacteur est ensuite introduit dans le four micro-onde Synthewave 402 (marque Prolabo, groupe Merck-Eurolab) muni d'un système d'agitation à pales. Le mélange est irradié pendant de 15 minutes à 90 minutes à une température et à une puissance appropriée. Après retour à la température ambiante, le mélange réactionnel est ensuite concentré à l'évaporateur rotatif. Au résidu d'évaporation on ajoute de l'éthanol (1 mL/gr. de produit). Après trituration du mélange dans l'éthanol, le solide insoluble est filtré sur verre fritté de porosité N°4 puis séché sous pression réduite. Celui-ci est éventuellement recristallisé dans l'éthanol.

**Mode opératoire au bain d'huile:** Une suspension constituée du (5Z)-5-arylidene-2-alkylthio-3,5-dihydroimidazol-4-one **4** (4 mmoles, 1 éq.) et d'amine aliphatique (40 mmoles, 10 éq.) est mélangée sous vive agitation magnétique et chauffée à une température inférieure de 10°C au point d'ébullition de cette amine (Tₑₓₚ. = Ebₐₘᵢₙₑ - 10°C) pendant 3 à 7 jours. Après retour à la température ambiante, les produits volatils sont éliminés sous pression réduite et on ajoute au milieu réactionnel de l'éther (~10 mL). Ensuite les produits insolubles dans l'éther sont collectés par filtration sur verre fritté de porosité n° 4. Le solvant résiduel du composé **5** est éliminé sous vide partiel dans un dessicateur pendant 2 heures, et on obtient la 2-amino-imidazolone **5** attendu sous la forme d'une poudre jaune.

### Exemple de 2-amino imidazolone 5:

### (5Z)-5-Benzo[1,3]dioxol-5-ylméthylène-3-méthyl-2 propylamino-3,5-dihydroimidazol-4-one (Ar¹ = 1,3-benzodioxol-5-yl, R¹ = Me, R³ = CH₂CH₂CH₃) :

Rdt: 48 %. Poudre jaune, mp = 190-192°C. RMN ¹H (300 MHz, CDCl₃) δ: 1,02 (t, 3H, J 7,4 Hz); 1,74 (sext, 2H, J 7,3 Hz, NHCH₂CH₂); 3,11 (s, 3H, NMe) ; 3,54 (t, 2H, *J* 6,2 Hz, NHCH₂C₂H₅); 4,95 (sl, 1H, NH); 5,98 (s, 2H); 6,62 (s, 1H, =CH) ; 6, 81 (d, 1H, J 8,1 Hz); 7, 34 (dd, 1H, J 8, 1; 1, 4 Hz); 7,99 (d, 1H, *J* 1,2 Hz). RMN ¹³C (75 MHz, CDCl₃) δ: 11,5 (qt, *J* 126; 4,0 Hz, NHC₂H₄Me); 22,8 (tq, J 135; 3,7 Hz, NHCH₂CH₂); 25,2 (q, *J* 140 Hz, NMe) ; 43,7 (tq, *J* 122; 7,0 Hz, NHCH₂); 101,1 (t, J 173 Hz, C-7'); 108,4 (d, *J* 164 Hz, C-2'); 110,3 (dt, J 166; 7,1 Hz, C-6); 116,8 (dt, J 157; 3,5 Hz); 126,1 (dt, *J* 163; 6,2 Hz, C-6'); 130,2 (d, J 7,8 Hz, C-5'); 138,1 (s, C-5); 147,6 (m, C-3'); 147,7 (m, C-4'); 157,2 (m, C-4); 170,4 (sm, C-2). SMHR, m/z: 287,1279 (calc. pour C₁₅H₁₇N₃O₃: 287,1270).

### REACTION F:

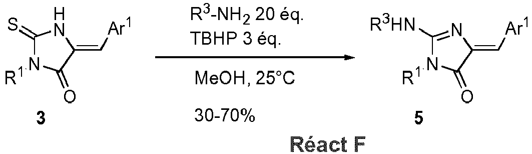

**Mode opératoire général:** A une solution de 3 (0.80 mmol) dans le MeOH (20 mL) est ajoutée 3 équivalents de *tertio*-Butyl hydroperoxyde TBHP (solution aqueuse à 70%) puis 20 équivalents d'amine. Le mélange réactionnel est agité à 25°C durant 3 jours. La solution est alors évaporée sous pression réduite et le résidu est purifié par flash-chromatographie sur gel de silice en utilisant comme éluant un mélange de CH₂Cl₂/MeOH (94/6).

### Exemple de composé 5:

### (5Z)-5-Benzo[1,3]dioxol-5-ylmethylene-2-ethylamino-3,5-dihydro-4H-imidazol-4-one (Ar¹ = 1,3-benzodioxol-5-yl , R³ = Et, R¹ = H).

Rdt = 40%. Poudre jaune, mp = 222-224°C. ¹H NMR (300 MHz, DMSO-d₆) : δ = 1.17 (t, *J =* 6.9 Hz, CH₃, 3H), 3.34 (m, CH₂, 2H), 6.02 (s, OCH₂O, 2H), 6.23 (s, =CH, 1H), 6.90 (d, *J* = 8.1 Hz, Hₐᵣ, 1H), 7.20 (br.S, NH, 1H), 7.38 (d, *J* = 8.1 Hz, Hₐᵣ, 1H), 7.93 (s, Hₐᵣ, 1H), 10.68 (br.s, NH, 1H). ¹³C NMR (75 MHz, DMSO-d₆) : δ = 15.5 (CH₃), 36.4 (NHCH₂), 101.4 (OCH₂O), 108.7, 109.8, 125.3, 131.0, 140.6, 146.9, 147.6, 160.2, 174.5. SMHR, m/z = 259.0959 (calculé pour C₁₃H₁₃N₃O₃ 259.0957).

### REACTION G:

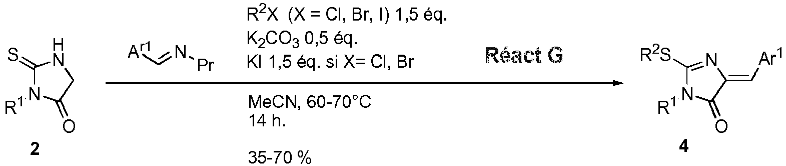

**Mode opératoire général:** Une suspension constituée de 5 mmoles d'aldimine Ar¹CH=N-Pr, 5 mmoles de thiohydantoïne **2** (R¹ = Me, Bu, Ph), 7,5 mmoles d'halogénoalcane R²X, 0,345 g (2,5 mmoles) de carbonate de potassium et éventuellement 1,25 g (7,5 mmoles) de KI (si utilisation d'un dérivé halogéné R²X avec X = Br ou Cl) dans 10 mL d'acétonitrile est chauffée pendant 14 heures à une température proche du point d'ébullition de l'halogénoalcane R²X (Tₑₓₚ. = Eb_{R2X} - 10°C). Le solvant de reaction est ensuite éliminé à l'évaporateur rotatif sous pression réduite. Le solide obtenu après évaporation est trituré avec du dichlorométhane (10 mL/gr. de produit) puis les sels inorganiques insolubles sont éliminés par filtration sur papier. Après évaporation du filtrat, le milieu réactionnel brut est traité (1 gr./10 mL) avec un mélange de pentane/éthanol (1/1). Le produit **4** attendu précipite, puis est collecté sur un verre fritté de porosité N°4 et séché au dessicateur sous vide partiel.

### Exemple de composé 4:

### (5Z)-5-(1,3-benzodioxol-5-ylméthylène)-3-méthyl-2-(éthylthio)-3,5-dihydro-4H-imidazol-4-one (Ar¹ = 1,3-benzodioxol-5-yl, R¹ = Me, R³ = Et) :

Rdt = 92%. Poudre jaune-orange, mp = 152-154°C. RMN ¹H (300 MHz, CDCl₃) δ : 1,55 (t, 3H, J = 7, 4Hz, SCH₂C**H**₃); 3,17 (s, 3H, NCH3); 3,40 (q, 2H, J = 7,4Hz, SC**H₂**CH₃); 6,00 (s, 2H, OCH₂O); 6,82 (d, 1H, J = 8,1 Hz, H-5); 6,83 (s, 1H, =CH); 7,37 (dd, 1H, J = 8,1 ; 1,0 Hz, H-6); 8,05 (s, 1H, H-2). RMN ¹³C (75 MHz, CDCl₃) δ : 14,7 (SCH₂**C**H₃); 25,6 (S**C**H₂CH₃); 26,9 (N**C**H₃); 101,8 (O**C**H₂O); 108,8 (C-5); 111,2 (C-2); 124,0 (=CH); 128,4 (C-6); 129,5 (C-1); 137,5 (N**C**=C); 148,3 (C-4); 149,5 (C-3); 164,1 (C-S); 170,3 (C=O). SMHR, *m*/*z =* 290,0730 trouvée (calculée pour C₁₄H₁₄N₂O₃S: 290,0725, M^{+.}).

### REACTION H:

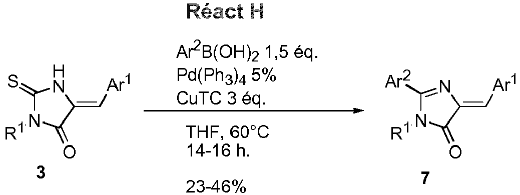

**Mode opératoire général:** Une solution constitué de (5Z)-5-arylidène thiohydantoïne **3** (1 équivalent), d'acide boronique Ar²B (OH)₂ (1,5 équivalent), de Pd(PPh₃)₄ (5 mol %) et de CuTC (3 équivalents) dans du THF anhydre (0,06 M) est introduite dans un tube de Schlenk. Le mélange réactionnel est porté au reflux du THF pendant une nuit sous vive agitation magnétique. Après retour à la température ambiante, le milieu de réaction est extrait avec du dichlorométhane (2 fois). La phase organique est lavée avec une solution d'hydrogénosulfate de sodium (1M), puis avec une solution de chlorure de sodium saturée et finalement avec une solution d'hydrogénocarbonate de sodium (1M). La phase organique est séchée sur MgSO₄, filtrée sur papier et le filtrat est concentré à l'évaporateur rotatif sous vide. Le résidu d'évaporation est mis en solution à chaud dans de l'éther diéthylique. Après refroidissement, les cristaux sont collectés par filtration sur verre fritté de porosité N°4 sous vide partiel puis purifiés par chromatographie sur gel de silice avec un mélange cyclohexane-acétate d'éthyle (70/30) comme éluant. La fraction de chromatographie est ensuite concentrée à l'évaporateur rotatif, séchée sous vide partiel et conduit au produit **7** attendu.

### Exemple de composé 7:

### (5Z)-5-(1,3-Benzodioxol-5-ylmethylene)-3-methyl-2-phenyl-3,5-dihydro-4H-imidazol-4-one (Ar¹ = 1,3-benzodioxol-5-yl, R¹ = Me, Ar² = C₆H₅) :

Rdt= 46%. Poudre jaune, mp = 209-211°C. RMN ¹H (300 MHz, CDCl₃) : δ = 3,35 (s, 3H, NCH₃) ; 6,01 (s, 2H, OCH₂O) ; 6,84 (d, 1H, *J* = 8,1 Hz, H-5') ; 7,16 (s, 1H, C=C**H**) ; 7,47 (dd, 1H, *J* = 8,1 Hz, J = 1,2 Hz, H-6'); 7,53 (m, 3H, H-3",H-4"); 7,84 (dd, 2H, *J* = 7,4 Hz, J = 2,2 Hz, H-2"); 8,14 (d, 1H, *J* = 1,2 Hz, H-2'). RMN ¹³C (75 MHz, CDCl₃) δ= 29,1 (NMe); 101,5 (OCH₂O); 108,5 (C-5'); 111,5 (C-2'); 128,7 (C-2"); 128,8 (C-3"); 128,8 (C=**C**H); 129,0 (C-6'); 129,4 (C-1"); 131,4 (C-4"); 137,5 (**C**=CH); 137,5 (C-1'); 148,1 (C-3'); 149,7 (C-4'); 161,4 (C=N); 171,6 (C=O). SMHR, *m*/*z* : 306,0995 (calculée pour C₁₈H₁₄N₂O₃ : 306,10044).

### Dosage de l'activité kinase de DYRK1A

### Réactifs biochimiques

ortho-vanadate de sodium, EGTA, Mops, ß-glycérophosphate, phénylphosphate, dithiothréitol (DTT), glutathione-agarose, glutathione, nitrophénylphosphate, protéine basique de la myéline ont été obtenus de Sigma Chemicals. [γ-³³P]-ATP proviennent d'Amersham.

### Préparation de la kinase DYRK1A et dosage enzymatique de son activité

La DYRK1A recombinante de rat a été exprimée chez *E. coli* comme protéine de fusion GST. Elle a été purifiée par chromatographie d'affinité sur billes de glutathion immobilisé (élution par du glutathion libre). L'activité kinase a été dosée dans le tampon C (60 mM ß-glycérophosphate, 15 mM p-nitrophénylphosphate, 25 mM Mops (pH 7,2), 5 mM EGTA, 15 mM MgCl₂, 1 mM DTT, 1 mM vanadate de sodium, 1 mM phénylphosphate), avec 1 mg protéine basique de la myéline /mL, en présence de 15 µM [γ-³³P] ATP (3, 000 Ci/mmol; 10 mCi/mL) dans un volume final de 30 µL. Apres 30 min d'incubation a 30°C, des aliquots 25 µL de surnageant ont été spottés sur des filtres Whatman P81 phosphocellulose et les filtres ont été lavés 5 fois dans une solution d'acide phosphorique (10 mL/L eau). La radioactivité incorporée dans le substrat, retenu sur les filtres humides, a été ensuite comptée en présence de liquide de scintillation ACS (Amersham). Les valeurs témoins ont été soustraites et les activités ont été exprimées en % de la valeur maximale, c'est-à-dire obtenue en l'absence d'inhibiteurs. Les valeurs d'IC₅₀ ont été calculées à partir de courbes dose-réponse et sont données en µM.

Les résultats sont donnés dans le tableau 2 ci-après :

**Tableau 2**

| | | | | |
|---|---|---|---|---|
| | | | | |
| | | | | |
| | | | | |
| | | | | |
| | | | | |
| | | | | |
| | | | | |
| | | | | |
| | | | | |
| | | | | |
| | | | | |
| | | | | |
| | | | | |
| | | | | |
| | | | | |
| | | | | |

### Références:

1: Microwave mediated solventless synthesis of new derivatives of marine alkaloid Leucettamine B. Jean René Chérouvrier, François Carreaux, Jean Pierre Bazureau Tetrahedron Letters 2002, 43, 3581-3584.
2: The insolation and synthesis of polyandrocarpamines A and B. Two new 2-aminoimidazolone compounds from the Füjian ascidian, Polyandrocarpa sp. Rohan A. Davis, William Aalbersberg, Semisi Meo, Rosan Moreira da Rocha, Chris M. Ireland Tetrahedron 2002, 58, 3263-3269.

| | | | | | |
|---|---|---|---|---|---|
| 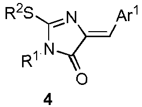 | | | | | |

| **Référence molécule** | **IC₅₀ DYRK1A (µM)** | **R¹** | **SR²** | **Ar¹** | **Réaction utilisée** |
|---|---|---|---|---|---|
| **ST099** | 6,8 | Me | SMe | 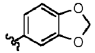 | **C** |
| **SB26** | 1,4 | Me | SEt | 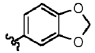 | **G** |
| **ST094** | 1,8 | Me | S*n*-Pr | 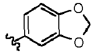 | **C** |
| **ST097** | 1,3 | Me | S*i*-Pr | 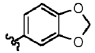 | **C** |
| **SB14** | 3,4 | Me | 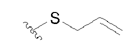 | 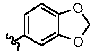 | **G** |
| **SB16 (invention)** | 0,9 | Me | 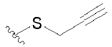 | 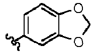 | **G** |
| **ST211 (invention)** | 0,47 | Me | 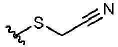 | 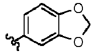 | **C** |
| **ST381 (invention)** | 0,44 | Me | SCH₂CH₂Cl | 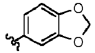 | **C** |
| **ST101** | 2,3 | Me | S*n*-Bu | 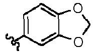 | **C** |
| **ST102** | 1,5 | Me | 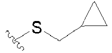 | 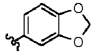 | **C** |
| **SA197** | 2,5 | Me | 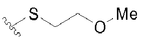 | 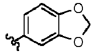 | **C** |
| **JR404** | 5,8 | Me | 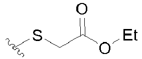 | 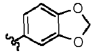 | **C** |
| **SB28** | 5,7 | Me | SBn | 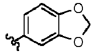 | **G** |
| **ST342** | >10 | Me | SEt | 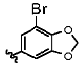 | **C** |
| **ST091** | >10 | Me | SEt | 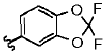 | **C** |
| **ST078** | 6,7 | Me | SMe | 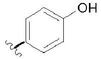 | **C** |
| **JR159** | >10 | Me | SMe | 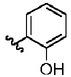 | **C** |
| **ST170** | >10 | Me | SEt | 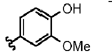 | **C** |
| **SB55** | >10 | Me | SEt | 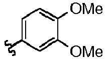 | **G** |
| **JR161** | >10 | Me | SMe | 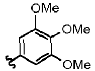 | **C** |
| **SB56** | >10 | Me | SEt | 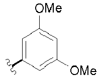 | **G** |
| **JR158** | >10 | Me | SMe | 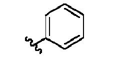 | **C** |
| **JR160** | >10 | Me | SMe | 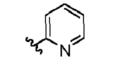 | **C** |
| **JR162** | >10 | Me | SMe | 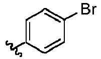 | **C** |
| **FB08** | >10 | Me | SEt | 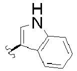 | **C** |
| **FB14** | >10 | Me | S*n*-Pr | 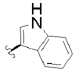 | **C** |
| **FB17** | >10 | Me | S*n*-Bu | 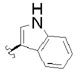 | **C** |
| **JR448** | >10 | *n*-Bu | SMe | 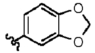 | **C** |
| **SB05** | >10 | *n*-Bu | SEt | 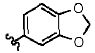 | **G** |
| **SB25** | >10 | *n*-Bu | 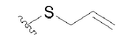 | 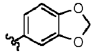 | **G** |
| **SB22** | >10 | *n*-Bu | 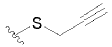 | 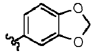 | **G** |
| **SB10** | >10 | *n*-Bu | SBn | 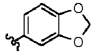 | **G** |
| **SB60** | >10 | *n*-Bu | 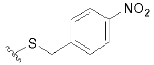 | 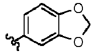 | **G** |
| **SB80** | >10 | *n*-Bu | SEt | 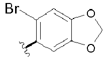 | **G** |
| **SB58** | >10 | *n*-Bu | SEt | 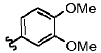 | **G** |
| **JR411** | >10 | Ph | 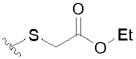 | 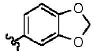 | **C** |
| **ST105 (invention)** | 0,68 | H | SMe | 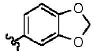 | **C** |
| **ST120 (invention)** | 0,44 | H | SEt | 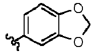 | **C** |
| **ST122 (invention)** | 0,44 | H | S*n*-Pr | 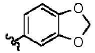 | **C** |
| **ST135 (invention)** | 0,46 | H | S*i*-Pr | 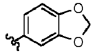 | **C** |
| **ST209 (invention)** | 0,17 | H | 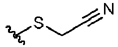 | 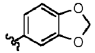 | **C** |
| **ST124 (invention)** | 0,59 | H | S*n*-Bu | 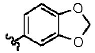 | **C** |
| **ST164 (invention)** | 0,65 | H | 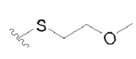 | 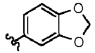 | **C** |
| **ST130 (invention)** | 0,5 | H | 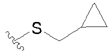 | 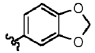 | **C** |
| **ST142 (invention)** | 0,78 | H | 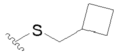 | 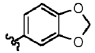 | **C** |
| **ST240** | 2,6 | H | SEt | 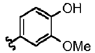 | **C** |
| **ST286** | >10 | H | SEt | 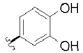 | **C** |
| **ST280** | >10 | H | SEt | 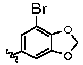 | **C** |

| | | | | | |
|---|---|---|---|---|---|
| 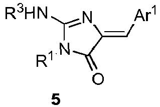 | | | | | |

| **Référence molécule** | **IC₅₀ DYRK1A (µM)** | **R¹** | **NHR³** | **Ar¹** | **Réaction utlisée** |
|---|---|---|---|---|---|
| **FC077** | 4,7 | Me | NH₂ | 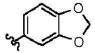 | **F** |
| **FC084** | 2,7 | Me | NHMe | 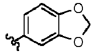 | **F** |
| **FC088 (invention)** | 0,9 | Me | NHEt | 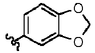 | **F** |
| **FCJR405** | 2,3 | Me | NH*n*-Pr | 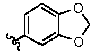 | **D** Bain d'huile |
| **FCJR232** | 1,3 | Me | NH*i*-Pr | 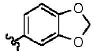 | **D** Bain d'huile |
| **FCFD13** | 1,6 | Me | 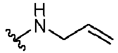 | 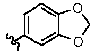 | **D** Bain d'huile |
| **MADE40 (inventio** | 0,73 | Me | NHCH₂CH₂OH | 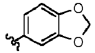 | **D** Micro-onde |
| **n)** | | | | | |
| **MADE26** | >10 | Me | 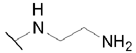 | 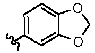 | **D** Micro-onde |
| **MADE47 (invention)** | **0,79** | Me | 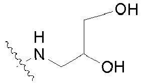 | 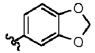 | **D** Micro-onde |
| **MADE24** | >10 | Me | 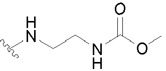 | 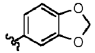 | **D** Micro-onde |
| **IA24** | >10 | Me | NHAc | 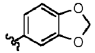 | **E** |
| **FCJR457** | 1,8 | Me | NH*n*-Bu | 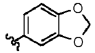 | **D** Bain d'huile |
| **FCFD24** | >10 | Me | NH*i*-Bu | 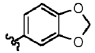 | **D** Bain d'huile |
| **FC107 (invention)** | 0,98 | Me | 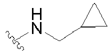 | 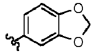 | **F** |
| **SA142** | 1,3 | Me | 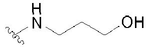 | 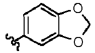 | **D** Bain d'huile |
| **FC103** | 1,2 | Me | 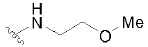 | 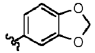 | **F** |
| **ST025** | 1,7 | Me | NH*i*-Am | 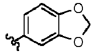 | **D** Bain d'huile |
| **MADE23** | 3 | Me | 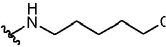 | 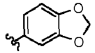 | **D** Micro-onde |
| **MADE10** | 1,4 | Me | 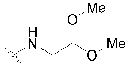 | 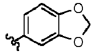 | **D** Micro-onde |
| **MADE9 (invention)** | 0,69 | Me | 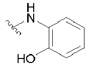 | 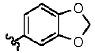 | **D** Micro-onde |
| | | | | | |
| **MADE8 (invention)** | 0,38 | Me | 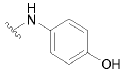 | 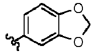 | **D** Micro-onde |
| **MADE33 (invention)** | 0,94 | Me | 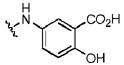 | 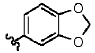 | **D** Micro-onde |
| **MADE30 (invention)** | 0,37 | Me | 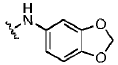 | 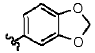 | **D** Micro-onde |
| **MADE29 (inventio n)** | 0,19 | Me | 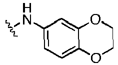 | 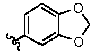 | **D** Micro-onde |
| **MADE39 (invention)** | 0,27 | Me | 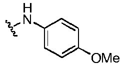 | 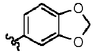 | **D** Micro-onde |
| **MADE37 (invention)** | 0,23 | Me | 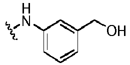 | 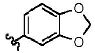 | **D** Micro-onde |
| **MADE36 (invention)** | 0,26 | Me | 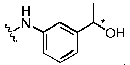 | 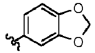 | **D** Micro-onde |
| | | | racémique | | |
| **MADE42** (invention) | 0,43 | Me | 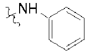 | 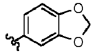 | **D** Micro-onde |
| | | | | | |
| **MADE34 (invention)** | 0,86 | Me | 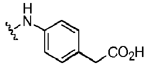 | 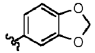 | **D** Micro-onde |
| **FC097** | 1,4 | Me | NHBn | 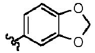 | **F** |
| **MADE12** | 4,6 | Me | 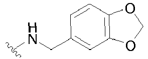 | 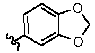 | **D** Micro-onde] |

| | | | | | |
|---|---|---|---|---|---|
| | | | | | |
| | | | | | |
| | | | | | |
| | | | | | |
| | | | | | |
| | | | | | |
| | | | | | |

| | | | | | |
|---|---|---|---|---|---|
| 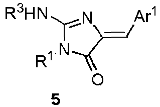 | | | | | |

| **Molécule référence** | **IC₅₀ DYK1A (µM)** | **R¹** | **NHR³** | **Ar¹** | **Réaction utilisée** |
|---|---|---|---|---|---|
| **ST092** | >10 | Me | **NH*n*-Pr** | 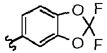 | **D** Bain d'huile |
| **FCFD14** | >10 | Me | NH*n*-Pr | 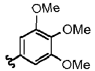 | **D** Bain d'huile |
| **FC104b** | >10 | Me | NH*i*-Pr | 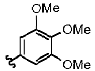 | **D** Bain d'huile |
| **FCFD11** | >10 | Me | NH*n*-Pr | 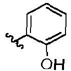 | **D** Bain d'huile |
| **FCFD08** | >10 | Me | NH*n*-Pr | 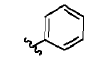 | **D** Bain d'huile |
| **FC095** | >10 | Me | NH*i*-Pr | 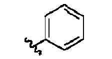 | **D** Bain d'huile |
| **FC092** | >10 | Me | NH*n*-Pr | 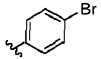 | **D** Bain d'huile |
| **FC109** | >10 | Me | NH*i*-Pr | 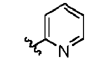 | **D** Bain d'huile |
| **SA164** | >10 | *n*-Bu | 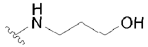 | 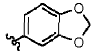 | **D** Micro-onde |
| **JR442** | >10 | *n*-Bu | NH*n*-Pr | 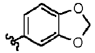 | **D** Bain d'huile |
| **FCJR464** | >10 | *n*-Bu | NH*n*-Bu | 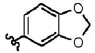 | **D** Bain d'huile |
| **JR445** | >10 | Ph | NH*n*-Pr | 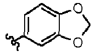 | **D** Bain d'huile |
| **FC085** | 1,7 | H | NHMe | 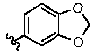 | **F** |
| **FC090** | 1,1 | H | NHEt | 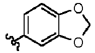 | **F** |
| **FC126 (invention)** | 0,89 | H | NH*n*-Pr | 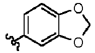 | **F** |
| **MADE44** (invention ) | 0,071 | H | 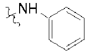 | 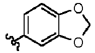 | **D** Micro-onde |
| **MADE48** (invention ) | 0,084 | H | 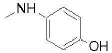 | 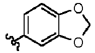 | **D** Micro-onde |
| **FC114 (invention)** | 0,5 | H | 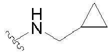 | 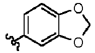 | **F** |
| **ST325 (invention)** | 0,17 | H | NH₂ | 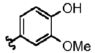 | **F** |
| **ST326** | >10 | H | NH₂ | 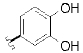 | **F** |
| **ST033** | 0.3 | Me | 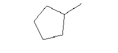 | 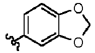 | **F** |

| | | | | | |
|---|---|---|---|---|---|
| 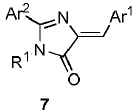 | | | | | |

| **Molécule référence** | **IC₅₀ DYRK1A (µM)** | **R¹** | **Ar²** | **Ar¹** | **Réaction utilisée** |
|---|---|---|---|---|---|
| **NL39** | 3,2 | Me | Ph | 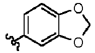 | **H** |
| **NL96 (invention)** | 0,43 | Me | C₆H₅-pOH | 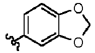 | **H** |
| **NL88A (invention)** | 0,22 | Me | 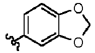 | 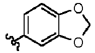 | **H** |

### Références:

1: Microwave mediated solventless synthesis of new derivatives of marine alkaloid Leucettamine B. Jean René Chérouvrier, François Carreaux, Jean Pierre Bazureau Tetrahedron Letters 2002, 43, 3581-3584.
2: Parallel solution phase synthesis of 2-alkylthio-3,5-dihydro-4H-imidazol-4-one by one-pot three component domino reaction. Stéven Renault, Sarah Bertrand, François Carreaux*, Jean Pierre Bazureau*. Journal of Combinatorial Chemistry 2007, 9, acceptée pour publication (attente d'autorisation pour ACS en ASAP).
3: Synthesis of the marine alkaloid Leucettamine B Nathalie Roué, Ian Bergman Tetrahedron 1999, 55, 14729-14738.
4: The isolation and synthesis of polyandrocarpamines A and B. Two new 2-aminoimidazalone compounds from the Fidjian ascidian, Polyandrocarpa sp. Rohan A. Davis, William Aalbersberg, Semisi Meo, Rosan Moreira da Rocha, Chris M. Ireland Tetrahedron 2002, 58, 3263-3269.

## Revendications

1. Dérivés d'imidazolones de formule (I) 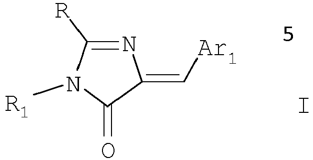 pour leur utilisation dans le traitement de maladies neurodégénératives, de la maladie de Pick et de la trisomie 21, choisis parmi les dérivés de formule (I) dans lesquels
**R=R₂S**
R₂= CH₂C≡CH; R₁ = CH₃; Ar₁ = 1,3-benzodioxol-5-yl,
R₂= CH₂C≡N; R₁ = CH₃; Ar₁ = 1,3-benzodioxol-5-yl,
R₂= CH₂CH₂Cl; R₁ = Me; Ar₁ = 1,3-benzodioxol-5-yl,
R₂= CH₃; R₁ = H; Ar₁ = 1,3-benzodioxol-5-yl,
R₂= CH₂CH₃; R₁ = H; Ar₁ = 1,3-benzodioxol-5-yl,
R₂= CH₂CH₂CH₃; R₁ = H; Ar₁ = 1,3-benzodioxol-5-yl,
R₂= CH(CH₃)₂; R₁ = H; Ar₁ = 1,3-benzodioxol-5-yl,
R₂= CH₂C≡N; R₁ = H; Ar₁ = 1,3-benzodioxol-5-yl,
R₂= CH₂CH₂CH₂CH₃; R₁ = H; Ar₁ = 1,3-benzodioxol-5-yl,
R₂= CH₂CH₂OCH₃; R₁ = H; Ar₁ = 1,3-benzodioxol-5-yl,
R₂= CH₂T1 avec T1 = cyclopropyl; R₁ = H; Ar₁ = 1,3-benzodioxol-5-yl,
R₂= CH₂T1 avec Tl= cyclobutyl; R₁ = H; Ar₁ = 1,3-benzodioxol-5-yl,
**R=R₃NH**
R₃= CH₂CH₃; R₁ = CH₃; Ar₁ = 1,3-benzodioxol-5-yl,
R₃= CH₂CH₂OH; R₁ = CH₃; Ar₁ = 1,3-benzodioxol-5-yl,
R₃= CH₂CH(OH)(CH₂OH); R₁ = CH₃; Ar₁ = 1,3-benzodioxol-5-yl,
R₃= CH₂T1 avec T1 = cyclopropyl; R₁ = CH₃; Ar₁ = 1,3-benzodioxol-5-yl,
R₃= o-OH-C₆H₄; R₁ = CH₃; Ar₁ = 1,3-benzodioxol-5-yl,
R₃= p-OH-C₆H₄; R₁ = CH₃; Ar₁ = 1,3-benzodioxol-5-yl,
R₃= p-OH,m-CO₂H-C₆H₄; R₁ = CH₃; Ar₁ = 1,3-benzodioxol-5-yl,
R₃= 1,3-benzodioxol-5-yl; R₁ = CH₃; Ar₁ = 1,3-benzodioxol-5-yl,
R₃= p-m-OCH₂CH₂O-C₆H₃; R₁ = CH₃; Ar₁ = 1,3-benzodioxol-5-yl,
R₃= p-OCH₃-C₆H₄; R₁ = CH₃; Ar₁ = 1,3-benzodioxol-5-yl,
R₃= m-CH₂OH-C₆H₄; R₁ = CH₃; Ar₁ = 1,3-benzodioxol-5-yl,
R₃= m-HOCH(CH₃)-C₆H₄; R₁ = CH₃; Ar₁ = 1,3-benzodioxol-5-yl,
R₃= C₆H₅; R₁ = CH₃; Ar₁ = 1,3-benzodioxol-5-yl,
R₃= p-CH₂COOH-C₆H₄; R₁ = CH₃; Ar₁ = 1,3-benzodioxol-5-yl,
R₃= CH₂CH₂CH₃; R₁ = H; Ar₁ = 1,3-benzodioxol-5-yl,
R₃= -C₆H₅; R₁ = H; Ar₁ = 1,3-benzodioxol-5-yl,
R₃= -p-OH-C₆H₄; R₁ = H; Ar₁ = 1,3-benzodioxol-5-yl,
R₃= CH₂T1 avec T1 = cyclopropyl; R₁ = H; Ar₁ = 1,3-benzodioxol-5-yl,
R₃= H; R₁ = H; Ar₁ = p-OH,m-OCH₃-C₆H₃,
**R=Ar₂**
Ar₂= -p-OH-C₆H₄; R₁ = CH₃; Ar₁ = 1,3-benzodioxol-5-yl,
Ar₂= 1,3-benzodioxol-5-yl; R₁ = CH₃; Ar₁ = 1,3-benzodioxol-5-yl.

2. Dérivés selon la revendication 1 pour leur utilisation dans le traitement de la maladie d'Alzheimer.

3. Dérivés d'imidazolones de formule (I) 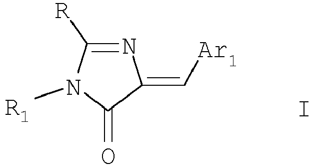 choisis parmi les dérivés dans lesquels
**R=R₂S**
R₂= CH₂C≡N; R₁ = CH₃; Ar₁ = 1,3-benzodioxol-5-yl,
R₂= CH₂CH₂Cl, R₁ = Me; Ar₁ = 1,3-benzodioxol-5-yl,
R₂= CH₃; R₁ = H; Ar₁ = 1,3-benzodioxol-5-yl,
R₂= CH₂CH₃; R₁ = H; Ar₁ = 1,3-benzodioxol-5-yl,R₂= CH₂CH₂CH₃; R₁ = H; Ar₁ = 1,3-benzodioxol-5-yl,
R₂= CH(CH₃)₂; R₁ = H; Ar₁ = 1,3-benzodioxol-5-yl,
R₂= CH₂C≡N; R₁ = H; Ar₁ = 1,3-benzodioxol-5-yl,
R₂= CH₂CH₂CH₂CH₃; R₁ = H; Ar₁ = 1,3-benzodioxol-5-yl,
R₂= CH₂CH₂OCH₃; R₁ = H; Ar₁ = 1,3-benzodioxol-5-yl,
R₂= CH₂T1 avec T1 = cyclopropyl; R₁ = H; Ar₁ = 1,3-benzodioxol-5-yl,
R₂= CH₂T1 avec Tl= cyclobutyl; R₁ = H; Ar₁ = 1,3-benzodioxol-5-yl,
**R=R₃NH**
R₃= CH₂CH₃; R₁ = CH₃; Ar₁ = 1,3-benzodioxol-5-yl,
R₃= CH₂CH₂OH; R₁ = CH₃; Ar₁ = 1,3-benzodioxol-5-yl,
R₃= CH₂CH(OH)(CH₂OH); R₁ = CH₃; Ar₁ = 1,3-benzodioxol-5-yl,
R₃= CH₂T1 avec T1 = cyclopropyl; R₁ = CH₃; Ar₁ = 1,3-benzodioxol-5-yl,
R₃= o-OH-C₆H₄; R₁ = CH₃; Ar₁ = 1,3-benzodioxol-5-yl,
R₃= p-OH-C₆H₄; R₁ = CH₃; Ar₁ = 1,3-benzodioxol-5-yl,
R₃= p-OH,m-CO₂H-C₆H₄; R₁ = CH₃; Ar₁ = 1,3-benzodioxol-5-yl,
R₃= 1,3-benzodioxol-5-yl; R₁ = CH₃; Ar₁ = 1,3-benzodioxol-5-yl,
R₃= p-m-OCH₂CH₂O-C₆H₃; R₁ = CH₃; Ar₁ = 1,3-benzodioxol-5-yl,
R₃= p-OCH₃-C₆H₄; R₁ = CH₃; Ar₁ = 1,3-benzodioxol-5-yl,
R₃= m-CH₂OH-C₆H₄; R₁ = CH₃; Ar₁ = 1,3-benzodioxol-5-yl,
R₃= m-HOCH(CH₃)-C₆H₄; R₁ = CH₃; Ar₁ = 1,3-benzodioxol-5-yl,
R₃= C₆H₅; R₁ = CH₃; Ar₁ = 1,3-benzodioxol-5-yl,
R₃= p-CH₂COOH-C₆H₄; R₁ = CH₃; Ar₁ = 1,3-benzodioxol-5-yl,
R₃= CH₂CH₂CH₃; R₁ = H; Ar₁ = 1,3-benzodioxol-5-yl,
R₃= -C₆H₅; R₁ = H; Ar₁ = 1,3-benzodioxol-5-yl,
R₃= -p-OH-C₆H₄; R₁ = H; Ar₁ = 1,3-benzodioxol-5-yl,
R₃= CH₂T1 avec T1 = cyclopropyl; R₁ = H; Ar₁ = 1,3-benzodioxol-5-yl,
**R=Ar₂**
Ar₂= -p-OH-C₆H₄; R₁ = CH₃; Ar₁ = 1,3-benzodioxol-5-yl,
Ar₂= 1,3-benzodioxol-5-yl; R₁ = CH₃; Ar₁ = 1,3-benzodioxol-5-yl.

4. Compositions pharmaceutiques, **caractérisées en ce qu'**elles renferment une quantité thérapeutiquement efficace d'au moins un dérivé selon la revendication précédente.

5. Compositions pharmaceutiques selon la revendication 4, pour le traitement de maladies neurodégénératives, en particulier de la maladie d'Alzheimer.

6. Compositions pharmaceutiques selon la revendication 4, pour le traitement de la maladie de Pick.

7. Compositions pharmaceutiques selon la revendication 4, pour le traitement de la trisomie 21.

## Patentansprüche

1. Imidazolon-Derivate mit der der Formel (I) 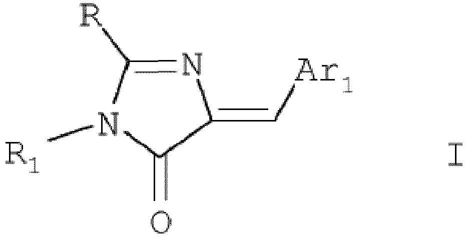 zur Verwendung als Arzneimittel zur Behandlung neurodegenerativer Krankheiten, der Pick-Krankheit und von Trisomie 21, ausgewählt aus Derivaten der Formel (I), wobei
**R=R₂S**
R₂= CH₂C≡CH; R₁ = CH₃; Ar₁ = 1,3-benzodioxol-5-yl,
R₂= CH₂C≡N; R₁ = CH₃; Ar₁ = 1,3-benzodioxol-5-yl,
R₂= CH₂CH₂C1; R₁ =Me; Ar₁ = 1,3-benzodioxol-5-yl,
R₂= CH₃; R₁ = H; Ar₁ = 1,3-benzodioxol-5-yl,
R₂= CH₂CH₃; R₁ = H; Ar₁ = 1,3-benzodioxol-5-yl,
R₂= CH₂CH₂CH₃; R₁ = H; Ar₁ = 1,3-benzodioxol-5-yl,
R₂= CH(CH₃)₂; R₁ = H; Ar₁ = 1,3-benzodioxol-5-yl,
R₂= CH₂C≡N; R₁ = H; Ar₁ = 1,3-benzodioxol-5-yl,
R₂= CH₂CH₂CH₂CH₃; R₁ = H; Ar₁ = 1,3-benzodioxol-5-yl,
R₂= CH₂CH₂0CH₃; R₁ = H; Ar₁ = 1,3-benzodioxol-5-yl,
R₂= CH₂Tl mit Tl= cyclopropyl; R₁ = H; Ar₁ = 1,3-benzodioxol-5-yl,
R₂= CH₂Tl mit Tl= cyclobutyl; R₁ = H; Ar₁ = 1,3-benzodioxol-5-yl
**R=R₃NH**
R₃= CH₂CH₃; R₁ = CH₃; Ar₁ = 1,3-benzodioxol-5-yl,
R₃= CH₂CH₂0H; R₁ = CH₃; Ar₁ = 1,3-benzodioxol-5-yl,
R₃= CH₂CH(OH) (CH₂0H); R₁ = CH₃; Ar₁ = 1,3-benzodioxol-5-yl,
R₃= CH₂Tl mit Tl= cyclopropyl; R₁ = CH₃; Ar₁ = 1,3-benzodioxol-5-yl,
R₃= o-OH-C₆H₄; R₁ = CH₃; Ar₁ = 1,3-benzodioxol-5-yl,
R₃= p-OH-C₆H₄; R₁ = CH₃; Ar₁ = 1,3-benzodioxol-5-yl,
R₃= p-OH, m-C0₂H-C₆H₄; R₁ = CH₃; Ar₁ = 1,3-benzodioxol-5-yl,
R₃= 1,3-benzodioxol-5-yl; R₁ = CH₃; Ar₁ = 1,3-benzodioxol-5-yl,
R₃= p-m-OCH₂CH₂0-C₆H₃; R₁ = CH₃; Ar₁ = 1,3-benzodioxol-5-yl,
R₃= p-OCH₃-C₆H₄; R₁ = CH₃; Ar₁ = 1,3-benzodioxol-5-yl,
R₃= m-CH₂0H-C₆H₄; R₁ = CH₃; Ar₁ = 1,3-benzodioxol-5-yl,
R₃= m-HOCH (CH₃) -C₆H₄; R₁ = CH₃; Ar₁ = 1,3-benzodioxol-5-yl,
R₃= C₆H₅; R₁ = CH₃; Ar₁ = 1,3-benzodioxol-5-yl,
R₃= p-CH₂COOH-C₆H₄; R₁ = CH₃; Ar₁ = 1,3-benzodioxol-5-yl,
R₃= CH₂CH₂CH₃; R₁ = H; Ar₁ = 1,3-benzodioxol-5-yl,
R₃= -C₆H₅; R₁ = H; Ar₁ = 1,3-benzodioxol-5-yl,
R₃= -p-OH-C₆H₄; R₁ = H; Ar₁ = 1,3-benzodioxol-5-yl,
R₃= CH₂Tl mit Tl= cyclopropyl; R₁ = H; Ar₁ = 1,3-benzodioxol-5-yl,
R₃= H; R₁ = H; Ar₁ = p-OH, m-OCH₃-C₆H₃,
**R=Ar₂**
Ar₂= -p-OH-C₆H₄; R₁ = CH₃; Ar₁ = 1,3-benzodioxol-5-yl,
Ar₂= 1,3-benzodioxol-5-yl; R₁ = CH₃; Ar₁ = 1,3-benzodioxol-5-yl.

2. Imidazolon-Derivate nach Anspruch 1 zur Verwendung bei der Behandlung der Alzheimer' schen Krankheit.

3. Imidazolon-Derivate mit der der Formel (I) 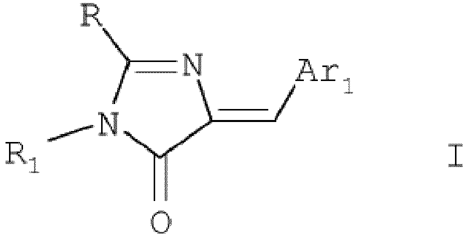 ausgewählt aus den Derivaten, in denen
**R=R₂S**
R₂= CH₂C≡CH; R₁ = CH₃; Ar₁ = 1,3-benzodioxol-5-yl,
R₂= CH₂C≡N; R₁ = CH₃; Ar₁ = 1,3-benzodioxol-5-yl,
R₂= CH₂CH₂Cl; R₁ =Me; Ar₁ = 1,3-benzodioxol-5-yl,
R₂= CH₃; R₁ = H; Ar₁ = 1,3-benzodioxol-5-yl,
R₂= CH₂CH₃; R₁ = H; Ar₁ = 1,3-benzodioxol-5-yl,
R₂= CH₂CH₂CH₃; R₁ = H; Ar₁ = 1,3-benzodioxol-5-yl,
R₂= CH(CH₃)₂; R₁ = H; Ar₁ = 1,3-benzodioxol-5-yl,
R₂= CH₂C≡N; R₁ = H; Ar₁ = 1,3-benzodioxol-5-yl,
R₂= CH₂CH₂CH₂CH₃; R₁ = H; Ar₁ = 1,3-benzodioxol-5-yl,
R₂= CH₂CH₂0CH₃; R₁ = H; Ar₁ = 1,3-benzodioxol-5-yl,
R₂= CH₂Tl mit Tl= cyclopropyl; R₁ = H; Ar₁ = 1,3-benzodioxol-5-yl,
R₂= CH₂Tl mit Tl= cyclobutyl; R₁ = H; Ar₁ = 1,3-benzodioxol-5-yl
**R=R₃NH**
R₃= CH₂CH₃; R₁ = CH₃; Ar₁ = 1,3-benzodioxol-5-yl,
R₃= CH₂CH₂0H; R₁ = CH₃; Ar₁ = 1,3-benzodioxol-5-yl,
R₃= CH₂CH (OH) (CH₂0H); R₁ = CH₃; Ar₁ = 1,3-benzodioxol-5-yl,
R₃= CH₂Tl mit Tl= cyclopropyl; R₁ = CH₃; Ar₁ = 1,3-benzodioxol-5-yl,
R₃= o-OH-C₆H₄; R₁ = CH₃; Ar₁ = 1,3-benzodioxol-5-yl,
R₃= p-OH-C₆H₄; R₁ = CH₃; Ar₁ = 1,3-benzodioxol-5-yl,
R₃= p-OH, m-C0₂H-C₆H₄; R₁ = CH₃; Ar₁ = 1,3-benzodioxol-5-yl,
R₃= 1,3-benzodioxol-5-yl; R₁ = CH₃; Ar₁ = 1,3-benzodioxol-5-yl,
R₃= p-m-OCH₂CH₂0-C₆H₃; R₁ = CH₃; Ar₁ = 1,3-benzodioxol-5-yl,
R₃= p-OCH₃-C₆H₄; R₁ = CH₃; Ar₁ = 1,3-benzodioxol-5-yl,
R₃= m-CH₂0H-C₆H₄; R₁ = CH₃; Ar₁ = 1,3-benzodioxol-5-yl,
R₃= m-HOCH (CH₃) -C₆H₄; R₁ = CH₃; Ar₁ = 1,3-benzodioxol-5-yl, R₃= C₆H₅; R₁ = CH₃; Ar₁ = 1,3-benzodioxol-5-yl,
R₃= p-CH₂COOH-C₆H₄; R₁ = CH₃; Ar₁ = 1,3-benzodioxol-5-yl,
R₃= CH₂CH₂CH₃; R₁ = H; Ar₁ = 1,3-benzodioxol-5-yl,
R₃= -C₆H₅; R₁ = H; Ar₁ = 1,3-benzodioxol-5-yl,
R₃= -p-OH-C₆H₄; R₁ = H; Ar₁ = 1,3-benzodioxol-5-yl,
R₃= CH₂Tl mit Tl= cyclopropyl; R₁ = H; Ar₁ = 1,3-benzodioxol-5-yl,
R₃= H; R₁ = H; Ar₁ = p-OH, m-OCH₃-C₆H₃,
**R=Ar₂**
Ar₂= -p-OH-C₆H₄; R₁ = CH₃; Ar₁ = 1,3-benzodioxol-5-yl,
Ar₂= 1,3-benzodioxol-5-yl; R₁ = CH₃; Ar₁ = 1,3-benzodioxol-5-yl.

4. Pharmazeutische Zusammensetzung, **dadurch gekennzeichnet, dass** sie eine therapeutisch wirksame Menge wenigstens eines Derivats nach dem vorhergehenden Anspruch enthält.

5. Pharmazeutische Zusammensetzung nach Anspruch 4 zur Behandlung neurodegenerativer Krankheiten, insbesondere der Alzheimer' schen Krankheit.

6. Pharmazeutische Zusammensetzung nach Anspruch 4 zur Behandlung der Pick-Krankheit.

7. Pharmazeutische Zusammensetzung nach Anspruch 4 zur Behandlung von Trisomie 21.

## Claims

1. Imidazolone derivatives of formula (I) 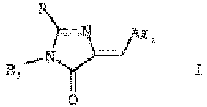 for use in the treatment of neurodegenerative diseases, Pick's disease and trisomy 21 syndrome, selected from the derivatives of formula (I) in which
**R=R₂S**
R₂= CH₂C=CH; R₁ = CH₃; Ar₁ = 1,3-benzodioxol-5-yl,
R₂= CH₂C≡N; R₁ = CH₃; Ar₁ = 1,3-benzodioxol-5-yl,
R₂= CH₂CH₂C1; R₁ = Me; Ar₁ = 1,3-benzodioxol-5-yl,
R₂= CH₃; R₁ = H; Ar₁ = 1,3-benzodioxol-5-yl,
R₂= CH₂CH₃; R₁ = H; Ar₁ = 1,3-benzodioxol-5-yl,
R₂= CH₂CH₂CH₃; R₁ = H; Ar₁ = 1,3-benzodioxol-5-yl,
R₂= CH(CH₃)₂; R₁ = H; Ar₁ = 1,3-benzodioxol-5-yl,
R₂= CH₂C≡N; R₁ = H; Ar₁ = 1,3-benzodioxol-5-yl,
R₂= CH₂CH₂CH₂CH₃; R₁ = H; Ar₁ = 1,3-benzodioxol-5-yl,
R₂= CH₂CH₂OCH₃; R₁ = H; Ar₁ = 1,3-benzodioxol-5-yl,
R₂= CH₂T1 with T1 = cyclopropyl; R₁ = H; Ar₁ - 1,3-benzodioxol-5-yl,
R₂= CH₂T1 with T1 = cyclobutyl; R₁ = H; Ar₁ = 1,3-benzodioxol-5-yl,
**R=R₃NH**
R₃= CH₂CH₃; R₁ = CH₃; Ar₁ = 1,3-benzodioxol-5-yl,
R₃= CH₂CH₂OH; R₁ = CH₃; Ar₁ = 1,3-benzodioxol-5-yl,
R₃= CH₂CH(OH)(CH₂OH); R₁ = CH₃; Ar₁ = 1,3-benzodioxol-5-yl,
R₃= CH₂T1 with T1 = cyclopropyl; R₁ = CH₃; Ar₁ = 1,3-benzodioxol-5-yl,
R₃= o-OH-C₆H₄; R₁ = CH₃; Ar₁ = 1,3-benzodioxol-5-yl,
R₃= p-OH-C₆H₄; R₁ = CH₃; Ar₁ = 1,3-benzodioxol-5-yl,
R₃= p-OH,m-CO₂H-C₆H₄; R₁ = CH₃; Ar₁ = 1,3-benzodioxol-5-yl,
R₃= 1,3-benzodioxol-5-yl; R₁ = CH₃; Ar₁ = 1,3-benzodioxol-5-yl,
R₃= p-m-OCH₂CH₂O-C₆H₃; R₁ = CH₃; Ar₁ = 1,3-benzodioxol-5-yl,
R₃= p-OCH₃-C₆H₄; R₁ = CH₃; Ar₁ = 1,3-benzodioxol-5-yl,
R₃= m-CH₂OH-C₆H₄; R₁ = CH₃; Ar₁ = 1,3-benzodioxol-5-yl,
R₃= m-HOCH(CH₃)-C₆H₄; R₁ = CH₃; Ar₁ = 1,3-benzodioxol-5-yl,
R₃= C₆H₅; R₁ = CH₃; Ar₁ = 1,3-benzodioxol-5-yl,
R₃= p-CH₂COOH-C₆H₄; R₁ = CH₃; Ar₁ = 1,3-benzodioxol-5-yl,
R₃= CH₂CH₂CH₃; R₁ = H; Ar₁ = 1,3-benzodioxol-5-yl,
R₃= -C₆H₅; R₁ = H; Ar₁ = 1,3-benzodioxol-5-yl,
R₃= -p-OH-C₆H₄; R₁ = H; Ar₁ = 1,3-benzodioxol-5-yl,
R₃= CH₂T1 with T1 = cyclopropyl; R₁ = H; Ar₁ = 1,3-benzodioxol-5-yl,
R₃= H; R₁ = H; Ar₁ = p-OH,m-OCH₃-C₆H₃,
**R=Ar₂**
Ar₂= -p-OH-C₆H₄; R₁ = CH₃; Ar₁ = 1,3-benzodioxol-5-yl,
Ar₂= 1,3-benzodioxol-5-yl; R₁ = CH₃; Ar₁ = 1,3-benzodioxol-5-yl.

2. Derivatives according to claim 1 for use in the treatment of Alzheimer's disease.

3. Imidazolone derivatives of formula (I) 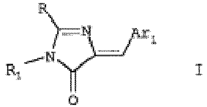 selected from the derivatives in which
**R=R₂S**
R₂= CH₂C≡N; R₁ = CH₃; Ar₁ = 1,3-benzodioxol-5-yl,
R₂= CH₂CH₂C1; R₁ = Me; Ar₁ = 1,3-benzodioxol-5-yl,
R₂= CH₃; R₁ = H; Ar₁ = 1,3-benzodioxol-5-yl,
R₂= CH₂CH₃; R₁ = H; Ar₁ = 1,3-benzodioxol-5-yl, R₂= CH₂CH₂CH₃; R₁ = H; Ar₁ = 1,3-benzodioxol-5-yl,
R₂ = CH(CH₃)₂; R₁ = H; Ar₁ = 1,3-benzodioxol-5-yl,
R₂= CH₂C≡N; R₁ = H; Ar₁ = 1,3-benzodioxol-5-yl,
R₂= CH₂CH₂CH₂CH₃; R₁ = H; Ar₁ = 1,3-benzodioxol-5-yl,
R₂= CH₂CH₂OCH₃; R₁ = H; Ar₁ = 1,3-benzodioxol-5-yl,
R₂= CH₂T1 with T1 = cyclopropyl; R₁ = H; Ar₁ = 1,3-benzodioxol-5-yl,
R₂= CH₂T1 with T1= cyclobutyl; R₁ = H; Ar₁ = 1,3-benzodioxol-5-yl,
**R=R₃NH**
R₃= CH₂CH₃; R₁ = CH₃; Ar₁ = 1,3-benzodioxol-5-yl,
R₃= CH₂CH₂OH; R₁ = CH₃; Ar₁ = 1,3-benzodioxol-5-yl,
R₃= CH₂CH(OH)(CH₂OH); R₁ = CH₃; Ar₁ = 1,3-benzodioxol-5-yl,
R₃= CH₂T1 with T1 = cyclopropyl; R₁ = CH₃; Ar₁ = 1,3-benzodioxol-5-yl,
R₃= o-OH-C₆H₄; R₁ = CH₃; Ar₁ = 1,3-benzodioxol-5-yl,
R₃= p-OH-C₆H₄; R₁ = CH₃; Ar₁ = 1,3-benzodioxol-5-yl,
R₃= p-OH,m-CO₂H-C6H₄; R₁ = CH₃; Ar₁ = 1,3-benzodioxol-5-yl,
R₃= 1,3-benzodioxol-5-yl; R₁ = CH₃; Ar₁ = 1,3-benzodioxol-5-yl,
R₃= p-m-OCH₂CH₂O-C6H₃; R₁ = CH₃; Ar₁ = 1,3-benzodioxol-5-yl,
R₃= p-OCH₃-C₆H₄; R₁ = CH₃; Ar₁ = 1,3-benzodioxol-5-yl,
R₃= m-CH₂OH-C6H₄; R₁ = CH₃; Ar₁ = 1,3-benzodioxol-5-yl,
R₃= m-HOCH(CH₃)-C6H₄; R₁ = CH₃; Ar₁ = 1,3-benzodioxol-5-yl,
R₃= C₆H₅; R₁ = CH₃; Ar₁ = 1,3-benzodioxol-5-yl,
R₃= p-CH₂COOH-C₆H₄; R₁ = CH₃; Ar₁ = 1,3-benzodioxol-5-yl,
R₃= CH₂CH₂CH₃; R₁ = H; Ar₁ = 1,3-benzodioxol-5-yl,
R₃= -C₆H₅; R₁ = H; Ar₁ = 1,3-benzodioxol-5-yl,
R₃= -p-OH-C₆H₄; R₁ = H; Ar₁ = 1,3-benzodioxol-5-yl,
R₃= CH₂T1 with T1 = cyclopropyl; R₁ = H; Ar₁ = 1,3-benzodioxol-5-yl,
**R=Ar₂**
Ar₂= -p-OH-C₆H₄; R₁ = CH₃; Ar₁ = 1,3-benzodioxol-5-yl,
Ar₂= 1,3-benzodioxol-5-yl; R₁ = CH₃; Ar₁ - 1,3-benzodioxol-5-yl.

4. Pharmaceutical compositions, **characterized in that** they contain a therapeutically effective amount of at least one derivative according to the preceding claim.

5. Pharmaceutical compositions according to claim 4, for treating neurodegenerative diseases, in particular Alzheimer's disease.

6. Pharmaceutical compositions according to claim 4, for treating Pick's disease.

7. Pharmaceutical compositions according to claim 4, for treating trisomy 21 syndrome.
